(19) Europäisches Patentamt
European Patent Office
Office européen des brevets

(11) EP 1 468 684 A1

(12) **EUROPEAN PATENT APPLICATION**
published in accordance with Art. 158(3) EPC

(43) Date of publication:
**20.10.2004 Bulletin 2004/43**

(21) Application number: **03701147.5**

(22) Date of filing: **22.01.2003**

(51) Int Cl.[7]: **A61K 31/445**, A61P 25/00, A61P 25/18, A61P 25/22, A61P 25/24, A61P 25/28, A61P 25/06, A61P 27/06, A61P 43/00, A61P 21/04, C07D 211/32

(86) International application number:
**PCT/JP2003/000553**

(87) International publication number:
**WO 2003/061658 (31.07.2003 Gazette 2003/31)**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **22.01.2002 JP 2002013362**
**22.01.2002 JP 2002013421**

(71) Applicant: **Eisai Co., Ltd.**
**Tokyo 112-8088 (JP)**

(72) Inventors:
- **IIMURA, Yoichi**
  **Tsukuba-shi, Ibaraki 305-0051 (JP)**
- **KOSASA, Takashi**
  **Tsukuba-gun, Ibaraki 300-2436 (JP)**
- **YAMANISHI, Yoshiharu**
  **Ryugasaki-shi, Ibaraki 301-0043 (JP)**

(74) Representative: **HOFFMANN EITLE**
**Patent- und Rechtsanwälte**
**Arabellastrasse 4**
**81925 München (DE)**

(54) **SIGMA RECEPTOR BINDER CONTAINING INDANONE DERIVATIVE**

(57) The present invention provides an indanone derivative and an excellent sigma receptor binding agent comprising an indanone derivative. More specifically, it provides a sigma receptor binding agent comprising an indanone derivative represented by the following formula, a pharmacologically acceptable salt thereof or a hydrate of them.

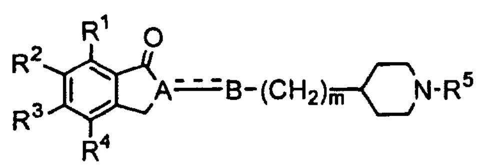

(I)

In the formula (I), $R^1$, $R^2$, $R^3$ and $R^4$ are the same as or different from each other and each represents hydrogen atom, a halogen atom, hydroxyl group, nitrile group, a $C_{1-6}$ alkyl group which may be substituted, a cycloalkyl group having three to eight carbon atoms which may be substituted, a $C_{1-6}$ alkoxy group which may be substituted, a cycloalkoxy group having three to eight carbon atoms which may be substituted, an acyl group having one to six carbon atoms which may be substituted, a $C_{1-6}$ alkoxycarbonyl group which may be substituted, a $C_{1-6}$ alkylaminocarbonyloxy group which may be substituted, a di($C_{1-6}$ alkyl)aminocarbonyloxy group which may be substituted, nitro group, an amino group which may be substituted, an amido group which may be substituted, mercapto group or a thio-$C_{1-6}$ alkoxy group which may be substituted, and further $R^1$ with $R^2$, $R^2$ with $R^3$, or $R^3$ with $R^4$ may together form an aliphatic ring, an aromatic ring, a heterocyclic ring or an alkylenedioxy ring; the partial structure:

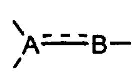

EP 1 468 684 A1

represents a group represented by >CH-$CH_2$-, >C=CH- or >C(-$R^7$)-$CH_2$-; m represents an integer of 0 or 1 to 5; and $R^5$ represents hydrogen atom, a $C_{1-6}$ alkyl group which may be substituted, a $C_{2-6}$ alkenyl group which may be substituted, a $C_{2-6}$ alkynyl group which may be substituted, a cycloalkyl group having three to eight carbon atoms which may be substituted, a 2,2-(alkylenedioxy)ethyl group or a group represented by the formula:

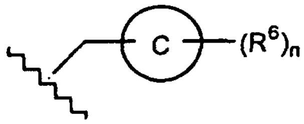

(wherein the ring C represents benzene ring, an aliphatic ring or a heterocyclic ring; $R^6$s are the same as or different from each other and each represents hydrogen atom, a halogen atom, hydroxyl group, nitrile group, a $C_{1-6}$ alkyl group which may be substituted, a $C_{2-6}$ alkenyl group which may be substituted, a $C_{2-6}$ alkynyl group which may be substituted, a cycloalkyl group having three to eight carbon atoms which may be substituted, a $C_{1-6}$ alkoxy group which may be substituted, a $C_{1-6}$ alkoxyalkoxy group which may be substituted, an aryloxy group which may be substituted or an aralkyloxy group which may be substituted, and further two of $R^6$s may together form an aliphatic ring, an aromatic ring, a heterocyclic ring or an alkylenedioxy ring; $R^7$ represents a halogen atom, hydroxyl group, a $C_{1-6}$ alkyl group which may be substituted, a $C_{1-6}$ alkoxy group, nitrile group, a halogeno-$C_{1-6}$ alkyl group, a hydroxyl-$C_{1-6}$ alkyl group, a cyano-$C_{1-6}$ alkyl group, an amino-$C_{1-6}$ alkyl group, nitro group, azide group, an amino group which may be substituted, a carbamoyl group which may be substituted, a carboxyl group which may be substituted, mercapto group or a thio-$C_{1-6}$ alkoxy group; and n represents an integer of 1 to 5), provided that 1-benzyl-4-[(5,6-dimethoxy-1-indanon)-2-yl]methylpiperidine, a pharmacologically acceptable salt thereof or a hydrate of them are excluded.

## Description

Technical Field

The present invention relates to an indanone derivative and a sigma receptor binding agent containing the indanone derivative.

Prior Art

Most of antipsychotic drugs and agents for treating schizophrenia conventionally clinically used are dopamine receptor antagonists. However, most of these antipsychotic drugs and agents for treating schizophrenia, such as haloperidol that is approved as effective in clinical use, show adverse drug actions such as extrapyramidal symptoms due to their dopamine receptor blocking action.

Recent studies have indicated that ligands and receptors belonging to non-dopaminergic mechanisms, such as serotonin, phencyclidine, muscarinic acetylcholine and sigma receptors, are also involved in mental disorders.

Among them, the sigma receptors were proposed as a subtype of opiate receptors that are combined typically with morphine to thereby induce hallucination by Martin et al., 1976. However, subsequent studies revealed that the sigma receptors are non-opiate receptors and that multitude of antipsychotic drugs and agents for treating schizophrenic disorder, such as haloperidol, have high affinity for the sigma receptors. Thus, compounds capable of binding to the sigma receptors have received attention as candidates for agents for treating schizophrenic disorder (Pharmacol. Reviews, 42, 355(1990)). Further studies on the sigma receptors have reported that compounds capable of binding to sigma receptors have a variety of actions, in addition to antipsychotic action, neuroprotection, antidepressant, anxiolytic, antidementia, anticonvulsive, drug dependency antagonistic, antiussive, stegnotic, anti-inflammatory, lacrimal fluid protein-release stimulating, and central micturition reflex depressant actions (Folia Pharmacol. Japon, 114, 3 (1999)).

Examples of compounds having a sigma receptor binding action can be found as 1-cycloalkylpiperidines, antipsychotic drugs, disclosed in JP-A 5-505172; sigma receptor antagonists disclosed in JP-A 6-329535; 1,4-(diphenylalkyl)piperazine derivatives disclosed in JP-A 7-89949; 2-arylalkenylazacycloalkane derivatives disclosed in JP-A 9-508893; and use for production of agents for treatment of a sigma-receptor-modulated disease disclosed in JP-A 11-503140.

J. Med. Chem., 37, 364(1994) and Neuroscience Lett., 260, 5(1999) disclose that 1-benzyl-4-[(5,6-dimethoxy-1-indanon)-2-yl]methylpiperidine, known as an acetylcholinesterase inhibitor, has a sigma receptor binding action. JP-A 11-349481 discloses that 1-benzyl-4-(5,6-dimethoxy-1-indanon)-2-yl]piperidine has a sigma receptor binding action.

The present inventors have reported the following compounds as compounds having an acetylcholinesterase inhibitory action in JP-B2 2733203.
A cyclic amine derivative represented by the following formula, a pharmacologically acceptable salt thereof or a hydrate of them.

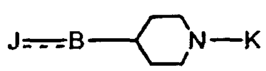

Wherein J is, for example, the formula:

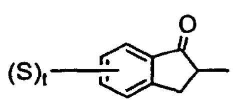

(wherein S is, for example, a lower alkoxy group having one to six carbon atoms; and t is 0 or an integer of 1 to 4); B is, for example, methylene chain; K is, for example, a benzyl group which may be substituted; and the partial structure:
-----
is a single bond or double bond, provided that a compound wherein J is 5,6-dimethoxy-1-indanon-2-yl group; B is $-CH_2-$ group; and K is unsubstituted benzyl group, a pharmacologically acceptable salt thereof or a hydrate of them are excluded.

However, the relationship between these compounds and the sigma receptors has not yet been known.

The present inventors have also reported the following compounds (1) to (3) as compounds having an acetylcholinesterase inhibitory action.

(1) A compound represented by the following formula:

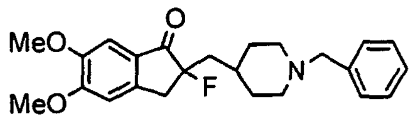

or a pharmacologically acceptable salt thereof (JP-A 2000-319257).

(2) A 4-substituted piperidine derivative fluoride represented by the following formula, a pharmacologically acceptable salt thereof or a hydrate of them (JP-A 2000-319258):

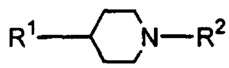

(wherein $R^1$ is, for example, a substituent represented by:

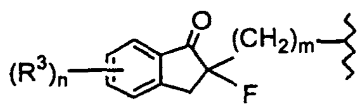

(wherein $R^3$s are the same as or different from each other and each represents, for example, a $C_{1-6}$ alkoxy group; m is 0 or an integer of 1 to 6; and n is an integer of 1 to 4); and $R^2$ is, for example, a benzyl group which may be substituted), provided that 1-benzyl-4-[(5,6-dimethoxy-2-fluoro-1-indanon)-2-yl]methylpiperidine or a pharmacologically acceptable salt thereof are excluded.

(3) A 4-substituted piperidine derivative represented by the following formula, a pharmacologically acceptable salt or a hydrate of them (JP-A 2001-139547):

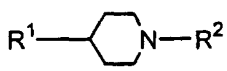

(wherein $R^1$ is, for example, a group represented by the formula:

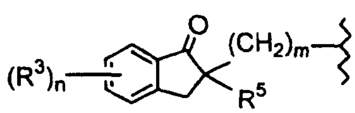

(wherein $R^3$s are the same as or different from each other and each represents, for example, a $C_{1-6}$ alkoxy group; $R^5$ is, for example, a halogen atom excluding fluorine atom; m is 0 or an integer of 1 to 6; and n is an integer of 1 to 4); and $R^2$ is, for example, a benzyl group which may be substituted).

However, the relationship between these compounds and the sigma receptors has not yet been known.

As is described above, compounds having a sigma receptor binding action are promising agents for treating various diseases, but conventional sigma receptor binding agents are now under development, and agents highly clinically usable have not yet been found. Therefore, demands have been made to provide sigma receptor binding agents having well-balanced efficacy and safety.

Disclosure of Invention

After intensive investigations to provide compounds and sigma receptor binding agents satisfying the above requirements, the present inventors have found that novel indanone derivatives have a sigma receptor binding action and are useful as sigma receptor binding agents. The present invention has been accomplished based on these findings.

Specifically, the present invention relates to:

1) a sigma receptor binding agent comprising an indanone compound represented by the formula:

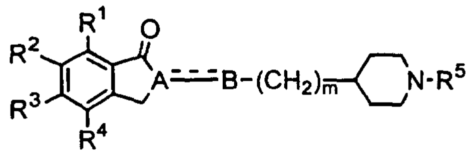

(I)

(in the formula (I), $R^1$, $R^2$, $R^3$ and $R^4$ are the same as or different from each other and each represents hydrogen atom, a halogen atom, hydroxyl group, nitrile group, a $C_{1-6}$ alkyl group which may be substituted, a cycloalkyl group having three to eight carbon atoms which may be substituted, a $C_{1-6}$ alkoxy group which may be substituted, a cycloalkoxy group having three to eight carbon atoms which may be substituted, a $C_{1-6}$ acyl group which may be substituted, a $C_{1-6}$ alkoxycarbonyl group having which may be substituted, a $C_{1-6}$ alkylaminocarbonyloxy group which may be substituted, a di($C_{1-6}$ alkyl)aminocarbonyloxy group which may be substituted, nitro group, an amino group which may be substituted, an amide group which may be substituted, mercapto group or a thio-$C_{1-6}$ alkoxy group which may be substituted, and further $R^1$ with $R^2$, $R^2$ with $R^3$, or $R^3$ with $R^4$ may together form an aliphatic ring, an aromatic ring, a heterocyclic ring or an alkylenedioxy ring; the partial structure:

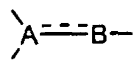

represents a group represented by >CH-$CH_2$-, >C=CH- or >C(-$R^7$)-$CH_2$-; m represents an integer of 0 or 1 to 5; and $R^5$ represents hydrogen atom, a $C_{1-6}$ alkyl group which may be substituted, a $C_{2-6}$ alkenyl group which may be substituted, a $C_{2-6}$ alkynyl group which may be substituted, a cycloalkyl group having three to eight carbon atoms which may be substituted, a 2,2-(alkylenedioxy)ethyl group or a group represented by the formula:

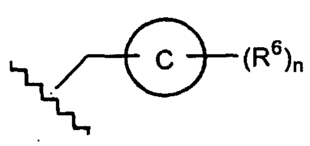

(wherein the ring C represents benzene ring, an aliphatic ring or a heterocyclic ring; $R^6$s are the same as or different from each other and each represents hydrogen atom, a halogen atom, hydroxyl group, nitrile group, a $C_{1-6}$ alkyl group which may be substituted, a $C_{2-6}$ alkenyl group which may be substituted, a $C_{2-6}$ alkynyl group which may be substituted, a cycloalkyl group having three to eight carbon atoms which may be substituted, a $C_{1-6}$ alkoxy group which may be substituted, a $C_{1-6}$ alkoxyalkoxy group which may be substituted, an aryloxy group which may be substituted or an aralkyloxy group which may be substituted, and further two of $R^6$s may together form an aliphatic ring, an aromatic ring, a heterocyclic ring or an alkylenedioxy ring; $R^7$ represents a halogen atom, hydroxyl group, a $C_{1-6}$ alkyl group, a $C_{1-6}$ alkoxy group, nitrile group, a halogeno-$C_{1-6}$ alkyl group, a hydroxyl-$C_{1-6}$ alkyl group, a cyano-$C_{1-6}$ alkyl group, an amino-$C_{1-6}$ alkyl group, nitro group, azide group, an amino group which may be substituted, carbamoyl group which may be substituted, carboxyl group which may be substituted, mercapto group or a thio-$C_{1-6}$ alkoxy group; and n represents an integer of 1 to 5), provided that 1-benzyl-4-[(5,6-dimethoxy-1-indanon)-2-yl]methylpiperidine, a pharmacologically acceptable salt thereof or a hydrate of them are excluded), a pharmacologically acceptable salt thereof or a hydrate of them; 2) the sigma receptor binding agent comprising an indanone compound, a pharmacologically acceptable salt thereof or a hydrate of them described in 1), wherein the indanone compound represented by the formula (I) is one selected from:

(1) 1-benzyl-4-[(5,6-dimethoxy-1-indanon)-2-ylidene]methylpiperidine,
(2) 1-benzyl-4-[(5,6-diethoxy-1-indanon)-2-ylidene]methylpiperidine,
(3) 1-benzyl-4-[(1-indanon)-2-yl]methylpiperidine,
(4) 1-benzyl-4-[(5-methoxy-1-indanon)-2-yl]methylpiperidine,
(5) 1-benzyl-4-[(5-ethoxy-6-methoxy-1-indanon)-2-yl]methylpiperidine,
(6) 1-benzyl-4-[(5,6-diethoxy-1-indanon)-2-yl]methylpiperidine,

(7) 1-benzyl-4-[[5,6-di(1-propyloxy)-1-indanon)-2-yl]methylpiperidine,
(8) 1-benzyl-4-[2-[(5,6-dimethoxy-1-indanon)-2-yl]ethyl]piperidine,
(9) 1-benzyl-4-[3-[(5,6-dimethoxy-1-indanon)-2-yl]propyl]piperidine,
(10) 1-(3-fluorobenzyl)-4-[(5,6-dimethoxy-1-indanon)-2-yl]methylpiperidine,
(11) 1-(3-methylbenzyl)-4-[(5,6-dimethoxy-1-indanon)-2-yl]methylpiperidine,
(12) 1-cyclohexylmethyl-4-[(5,6-dimethoxy-1-indanon)-2-yl]methylpiperidine,
(13) 1-benzyl-4-[(2-fluoro-1-indanon)-2-yl]methylpiperidine,
(14) 1-benzyl-4-[(5-methoxy-2-fluoro-1-indanon)-2-yl]methylpiperidine,
(15) 1-benzyl-4-[(5,6-dimethoxy-2-fluoro-1-indanon)-2-yl]methylpiperidine,
(16) 1-benzyl-4-[(5,6-diethoxy-2-fluoro-1-indanon)-2-yl]methylpiperidine,
(17) 1-benzyl-4-[[5,6-di(1-propyloxy)-2-fluoro-1-indanon]-2-yl]methylpiperidine,
(18) 1-benzyl-4-[(5,6-dimethoxy-2-fluoro-1-indanon)-2-yl]piperidine,
(19) 1-benzyl-4-[2-[(5,6-dimethoxy-2-fluoro-1-indanon)-2-yl]ethyl]piperidine,
(20) 1-benzyl-4-[3-[(5,6-dimethoxy-2-fluoro-1-indanon)-2-yl]propyl]piperidine,
(21) 1-(2-fluorobenzyl)-4-[(5,6-dimethoxy-2-fluoro-1-indanon)-2-yl]methylpiperidine,
(22) 1-(3-fluorobenzyl)-4-[(5,6-dimethoxy-2-fluoro-1-indanon)-2-yl]methylpiperidine,
(23) 1-(4-fluorobenzyl)-4-[(5,6-dimethoxy-2-fluoro-1-indanon)-2-yl]methylpiperidine,
(24) 1-(3-methylbenzyl)-4-[(5,6-dimethoxy-2-fluoro-1-indanon)-2-yl]methylpiperidine,
(25) 1-cyclohexylmethyl-4-[(5,6-dimethoxy-2-fluoro-1-indanon)-2-yl]methylpiperidine,
(26) 1-benzyl-4-[(5,6-dimethoxy-2-chloro-1-indanon)-2-yl]methylpiperidine,
(27) 1-benzyl-4-[(5,6-diethoxy-2-chloro-1-indanon)-2-yl]methylpiperidine,
(28) 1-benzyl-4-[(5-ethoxy-6-methoxy-2-chloro-1-indanon)-2-yl]methylpiperidine,
(29) 1-benzyl-4-[(5,6-dimethoxy-2-bromo-1-indanon)-2-yl]methylpiperidine, and
(30) 1-benzyl-4-[(5,6-dimethoxy-2-methyl-1-indanon)-2-yl]methylpiperidine; 3) the sigma receptor binding agent described in 1) or 2), which is a sigma receptor antagonist or a sigma receptor agonist; 4) the sigma receptor binding agent described in 1) or 2), which is an agent for preventing, treating or improving a disease against which a sigma receptor agonistic drug is efficacious; 5) the sigma receptor binding agent described in 1) or 2), which is an agent for preventing, treating or improving a disease against which a sigma receptor antagonistic action is efficacious; 6) the sigma receptor binding agent described in 1) or 2), which is an agent for preventing, treating or improving a disease against which a sigma receptor agonistic action is efficacious; 7) the sigma receptor binding agent described in 1) or 2), which is an agent for preventing, treating or improving a mental disorder; 8) the sigma receptor binding agent described in 7), wherein the mental disorder is at least one selected from a disorder accompanied with cerebrovascular dementia and/or senile dementia, schizophrenia, emotional disorder, depression, neurosis, psychophysiologic disorder and anxiety; 9) the sigma receptor binding agent described in 8), wherein the disorder accompanied with cerebrovascular dementia and/or senile dementia is at least one selected from aggressive behavior, mental excitement, wandering, delirium, hallucination and hyperkinesis; 10) the sigma receptor binding agent according to claim 1 or 2, which is an agent for improving intellectual function; 11) the indanone compound, a pharmacologically acceptable salt thereof or a hydrate of them, wherein the indanone compound represented by the formula (I) is one selected from:

(1) 1-benzyl-4-[[5,6-(1,2-ethylenedioxy)-1-indanon]-2-ylidene]methylpiperidine,
(2) 1-benzyl-4-[(5-cyclohexyl-1-indanon)-2-ylidene]methylpiperidine,
(3) 1-benzyl-4-[(5-cyclohexyloxy-6-methoxy-1-indanon)-2-ylidene]methylpiperidine,
(4) 1-benzyl-4-[[5-methoxy-6-(2-propyloxy)-1-indanon]-2-ylidene]methylpiperidine,
(5) 1-benzyl-4-[[5,6-(1,2-ethylenedioxy)-1-indanon]-2-yl]methylpiperidine,
(6) 1-benzyl-4-[[5,6-cyclohexyl-1-indanon]-2-yl]methylpiperidine,
(7) 1-benzyl-4-[(5-cyclohexyloxy-6-methoxy-1-indanon)-2-yl]methylpiperidine,
(8) 1-benzyl-4-[[5-methoxy-6-(2-propyloxy)-1-indanon]-2-yl]methylpiperidine,
(9) 1-benzyl-4-[(6-ethoxy-5-methoxy-1-indanon)-2-yl]methylpiperidine,
(10) 1-benzyl-4-[[6-methoxy-5-(1-propyloxy)-1-indanon]-2-yl]methylpiperidine,
(11) 1-benzyl-4-[(5-cyanomethoxy-6-methoxy-1-indanon)-2-yl]methylpiperidine,
(12) 1-cyclopentylmethyl-4-[(5,6-diethoxy-1-indanon)-2-yl]methylpiperidine,
(13) 1-cyclohexylmethyl-4-[(5,6-diethoxy-1-indanon)-2-yl]methylpiperidine,
(14) 1-cycloheptylmethyl-4-[(5,6-diethoxy-1-indanon)-2-yl]methylpiperidine,
(15) 1-cyclooctylmethyl-4-[(5,6-diethoxy-1-indanon)-2-yl]methylpiperidine,
(16) 1-(2-fluorobenzyl)-4-[(5,6-diethoxy-1-indanon)-2-yl]methylpiperidine,
(17) 1-(3-fluorobenzyl)-4-[(5,6-diethoxy-1-indanon)-2-yl]methylpiperidine,

(18) 1-(4-fluorobenzyl)-4-[(5,6-diethoxy-1-indanon)-2-yl]methylpiperidine,
(19) 1-(2-chlorobenzyl)-4-[(5,6-diethoxy-1-indanon)-2-yl]methylpiperidine,
(20) 1-(3-chlorobenzyl)-4-[(5,6-diethoxy-1-indanon)-2-yl]methylpiperidine,
(21) 1-(4-chlorobenzyl)-4-[(5,6-diethoxy-1-indanon)-2-yl]methylpiperidine,
(22) 1-(2-methylbenzyl)-4-[(5,6-diethoxy-1-indanon)-2-yl]methylpiperidine,
(23) 1-(3-methylbenzyl)-4-[(5,6-diethoxy-1-indanon)-2-yl]methylpiperidine,
(24) 1-(4-methylbenzyl)-4-[(5,6-diethoxy-1-indanon)-2-yl]methylpiperidine,
(25) 1-benzyl-4-[(6-ethoxy-5-methoxy-2-fluoro-1-indanon)-2-yl]methylpiperidine,
(26) 1-benzyl-4-[(5-ethoxy-6-methoxy-2-fluoro-1-indanon)-2-yl]methylpiperidine,
(27) 1-benzyl-4-[[6-methoxy-5-(1-propyloxy)-2-fluoro-1-indanon]-2-yl]methylpiperidine,
(28) 1-(2-fluorobenzyl)-4-[(5,6-diethoxy-2-fluoro-1-indanon)-2-yl]methylpiperidine,
(29) 1-(3-fluorobenzyl)-4-[(5,6-diethoxy-2-fluoro-1-indanon)-2-yl]methylpiperidine,
(30) 1-(4-fluorobenzyl)-4-[(5,6-diethoxy-2-fluoro-1-indanon)-2-yl]methylpiperidine,
(31) 1-(2-chlorobenzyl)-4-[(5,6-diethoxy-2-fluoro-1-indanon)-2-yl]methylpiperidine,
(32) 1-(3-chlorobenzyl)-4-[(5,6-diethoxy-2-fluoro-1-indanon)-2-yl]methylpiperidine,
(33) 1-(4-chlorobenzyl)-4-[(5,6-diethoxy-2-fluoro-1-indanon)-2-yl]methylpiperidine,
(34) 1-(2-methylbenzyl)-4-[(5,6-diethoxy-2-fluoro-1-indanon)-2-yl]methylpiperidine,
(35) 1-(3-methylbenzyl)-4-[(5,6-diethoxy-2-fluoro-1-indanon)-2-yl]methylpiperidine,
(36) 1-(4-methylbenzyl)-4-[(5,6-diethoxy-2-fluoro-1-indanon)-2-yl]methylpiperidine,
(37) 1-cyclopentylmethyl-4-[(5,6-diethoxy-2-fluoro-1-indanon)-2-yl]methylpiperidine,
(38) 1-cyclohexylmethyl-4-[(5,6-diethoxy-2-fluoro-1-indanon)-2-yl]methylpiperidine,
(39) 1-cycloheptylmethyl-4-[(5,6-diethoxy-2-fluoro-1-indanon)-2-yl]methylpiperidine,
(40) 1-cyclooctylmethyl-4-[(5,6-diethoxy-2-fluoro-1-indanon)-2-yl]methylpiperidine,
(41) 1-benzyl-4-[(5-cyanomethoxy-6-methoxy-2-fluoro-1-indanon)-2-yl]methylpiperidine,
(42) 1-(3,4-difluorobenzyl)-4-[(5,6-diethoxy-1-indanon)-2-yl]methylpiperidine,
(43) 1-(3,5-difluorobenzyl)-4-[(5,6-diethoxy-1-indanon)-2-yl]methylpiperidine,
(44) 1-(3,4-difluorobenzyl)-4-[(5,6-diethoxy-2-fluoro-1-indanon)-2-yl]methylpiperidine, and
(45) 1-(3,5-difluorobenzyl)-4-[(5,6-diethoxy-2-fluoro-1-indanon)-2-yl]methylpiperidine; 12) a pharmaceutical composition comprising the indanone compound described in 11), a pharmacologically acceptable salt thereof or a hydrate of them; 13) the pharmaceutical composition according to claim 12, which is a sigma receptor binding agent; 14) the pharmaceutical composition described in 12), which is a sigma receptor antagonist or a sigma receptor agonist; 15) the pharmaceutical composition described in 12), which is an agent for preventing, treating or improving a disease against which a sigma receptor-active drug is efficacious; 16) the pharmaceutical composition described in 12), which is an agent for preventing, treating or improving a disease against which a sigma receptor antagonistic action is efficacious; 17) the pharmaceutical composition described in 12), which is an agent for preventing, treating or improving a disease against which a sigma receptor agonistic action is efficacious; 18) the pharmaceutical composition described in 12), which is an agent for preventing, treating or improving a mental disorder; 19) the pharmaceutical composition described in 12), wherein the mental disorder is at least one selected from a disorder accompanied with cerebrovascular dementia and/or senile dementia, schizophrenia, emotional disorder, depression, neurosis, psychosomatic disorder and anxiety; 20) the pharmaceutical composition described in 19), wherein the disorder accompanied with cerebrovascular dementia and/or senile dementia is at least one selected from aggressive behavior, mental excitement, wandering, delirium, hallucination and hyperkinesis; 21) the pharmaceutical composition described in 12), which is an agent for improving intellectual function; 22) the pharmaceutical composition described in 12), which is an acetylcholinesterase inhibitor; 23) the pharmaceutical composition described in 12), which is an agent for preventing, treating or improving senile dementia, cerebrovascular dementia, attention-deficit hyperactivity disorder, glaucoma, myasthenia gravis or migraine; and 24) the pharmaceutical composition described in 23), wherein the senile dementia is Alzheimer-type dementia.

The present invention further provides a method for preventing, treating or improving a disease against which a sigma receptor binding action is efficacious, which comprises administering a pharmacologically effective amount of the indanone derivative represented by the formula (I), a pharmacologically acceptable salt thereof or a hydrate of them to a patient.

The present invention further provides use of the indanone derivative represented by the formula (I), a pharmacologically acceptable salt thereof or a hydrate of them, for producing an agent for preventing, treating or improving a disease against which a sigma receptor binding action is efficacious.

In addition, the present invention provides therapeutic use of the compound described in the above 11), a pharmaceutically acceptable salt thereof or hydrate of them.

Specifically, the present invention provides a method for preventing, treating or improving a disease against which a sigma receptor binding action is efficacious, which comprises administering a pharmacologically effective amount of the indanone derivative described in the above 11), a pharmacologically acceptable salt thereof or a hydrate of them to a patient.

The present invention also provides use of the indanone derivative described in the above 11), a pharmacologically acceptable salt thereof or a hydrate of them, for producing an agent for preventing, treating or improving a disease against which a sigma receptor binding action is efficacious.

The present invention also includes the therapeutic use of the compounds represented by the formula (I), except for the compound described in the above 11), a pharmacologically acceptable salt thereof or a hydrate of them.

The symbols, terms and other descriptions as used herein will be explained, and the present invention will be illustrated in detail below.

The "disease against which a sigma receptor binding action is efficacious" as used in the present invention includes a disease against which a sigma receptor antagonistic action is efficacious, and a disease against which a sigma receptor agonistic action is efficacious. Examples of the disease are mental disorders, intellectual dysfunction, diseases against which an acetylcholinesterase inhibitory action is efficacious, senile dementia including Alzheimer-type dementia, cerebrovascular dementia, attention-deficit hyperactive disorder, glaucoma, myasthenia gravis and migraine. Specific examples of the above-mentioned mental disorders are a disorder accompanied with cerebrovascular dementia and/or senile dementia, such as aggressive behavior, mental excitement, wandering, delirium, hallucination and/or hyperkinesis; as well as schizophrenia, emotional disorder, depression, neurosis, psychophysiologic disorder and anxiety.

The "halogen atom" as used in the formula (1) refers to, for example, fluorine atom, chlorine atom, bromine atom and iodine atom, of which fluorine atom, chlorine atom and bromine atom are preferred.

The "$C_{1-6}$ alkyl group" in the formula (I) means an alkyl group having one to six carbon atoms, and preferred examples are a linear or branched alkyl group such as methyl group, ethyl group, n-propyl group, i-propyl group, n-butyl group, i-butyl group, t-butyl group, n-pentyl group, i-pentyl group, neopentyl group, hexyl group, 1-methylpropyl group, 1-ethylpropyl group, 1-methylbutyl group or 2-methylbutyl group, of which methyl group, ethyl group, n-propyl group, i-propyl group, 2-methyl-1-propyl group and t-butyl group are more preferred.

The term "cycloalkyl group having three to eight carbon atoms" in the formula (I) means a cyclic alkyl group having three to eight carbon atoms, and preferred examples are cyclopropyl group, cyclobutyl group, cyclopentyl group, cyclohexyl group, cycloheptyl group and cyclooctyl group.

The "$C_{1-6}$ alkoxy group" in the formula (I) is preferably a linear or branched alkoxy group such as methoxy group, ethoxy group, n-propoxy group, i-propoxy group, n-butoxy group, i-butoxy group, t-butoxy group, pentyloxy group or hexyloxy group. Preferred examples of the "substituent" in the "$C_{1-6}$ alkoxy group which may be substituted" are hydroxyl group, a halogen atom, nitrile group and nitro group.

The "cycloalkoxy group having three to eight carbon atoms" in the formula (I) is preferably cyclopropoxy group, cyclobutoxy group, cyclopentyloxy group, cyclohexyloxy group, cycloheptyloxy group or cyclooctyloxy group, of which cyclopentyloxy group, cyclohexyloxy group, cycloheptyloxy group and cyclooctyloxy group are more preferred.

The "$C_{1-6}$ acyl group" in the formula (I) means a linear or branched acyl group derived from a fatty acid having one to six carbon atoms, and, for example, formyl group, acetyl group, propionyl group, butyryl group, isobutyryl group, valeryl group, isovaleryl group, pivaloyl group and hexanoyl group are preferred.

A suitable "$C_{1-6}$ alkoxy-carbonyl group" in the formula (I) is methoxycarbonyl group, ethoxycarbonyl group, n-propoxycarbonyl group, i-propoxycarbonyl group, n-butoxycarbonyl group, i-butoxycarbonyl group, tert-butoxycarbonyl group, pentyloxycarbonyl group or hexyloxycarbonyl group, of which methoxycarbonyl group, ethoxycarbonyl group, n-propoxycarbonyl group and i-propoxycarbonyl group are more preferred.

Suitable examples of the "$C_{1-6}$ alkyl-aminocarbonyloxy group" and "di($C_{1-6}$ alkyl)-aminocarbonyloxy group" in the formula (I) are methylaminocarbonyloxy group, ethylaminocarbonyloxy group, n-propylaminocarbonyloxy group, i-propylaminocarbonyloxy group, n-butylaminocarbonyloxy group, i-butylaminocarbonyloxy group, tert-butylaminocarbonyloxy group, n-pentylaminocarbonyloxy group, i-pentylaminocarbonyloxy group, neopentylaminocarbonyloxy group, hexylaminocarbonyloxy group, 1-methylpropylaminocarbonyloxy group, 1-methylbutylaminocarbonyloxy group, 2-methylbutylaminocarbonyloxy group, dimethylaminocarbonyloxy group, diethylaminocarbonyloxy group, di-(n-propyl)-aminocarbonyloxy group, di-(i-propyl)-aminocarbonyloxy group, di-(n-butyl)-aminocarbonyloxy group, di-(i-butyl)-aminocarbonyloxy group, di-(tert-butyl)-aminocarbonyloxy group, di-(n-pentyl)-aminocarbonyloxy group, di-(i-pentyl)-aminocarbonyloxy group, di-(neopentyl)-aminocarbonyloxy group, di-(n-hexyl)-aminocarbonyloxy group, di-(1-methylpropyl)-aminocarbonyloxy group, di-(1-methylbutyl)-aminocarbonyloxy group and di-(2-methylbutyl)-aminocarbonyloxy group.

The "amino group which may be substituted" in the formula (I) means an amino group whose nitrogen atom may be substituted with, for example, an alkyl group having one to six carbon atoms or sulfonic acid residue, and the "amino group" also includes a cyclic amino group. Examples of the "amino group which may be substituted" include

amino group, methylamino group, dimethylamino group, pyrrolidinyl group, pyrazolinyl group, piperidyl group, piperazinyl group, acetamide group, propionamide group, methanesulfonamide group, ethanesulfonamide group, toluenesulfonamide group and N-methylacetamide group.

The "amide group which may be substituted" in the formula (I) means an amide group which may be substituted with a group such as an alkyl group having one to six carbon atoms. The amide group herein also includes an amide group of a cyclic amine. Examples of the "amide group which may be substituted" are amide group, N-methylamide group, N,N-dimethylamide group, N-ethylamide group, N,N-diethylamide group, N-methyl-N-ethylamide group, pyrrolidinylcarbonyl group, pyrazolinylcarbonyl group, piperidylcarbonyl group and piperazinylcarbonyl group.

The "thio-$C_{1-6}$ alkoxy group" in the formula (I) means sulfur atom combined with a group having the same meaning as in the definition of the "$C_{1-6}$ alkyl group" and includes, for example, methylthio group ($-SCH_3$) or ethylthio group ($-SC_2H_5$).

In the formula (I), the "aliphatic ring" is not specifically limited, but is preferably cyclopentane ring, cyclohexane ring, cycloheptane ring or cyclooctane ring. A preferred "aromatic ring" is, for example, furan ring, thiophene ring, pyrrole ring, imidazole ring, oxazole ring, thiazole ring, triazole ring, pyridine ring, pyrazine ring, pyrimidine ring, tetrahydrofuran ring, tetrahydropyran ring, dioxane ring, dioxolane ring, piperidine ring, piperazine ring, morpholine ring or thiomorpholine ring.

A preferred example in the case when "$R^1$ with $R^2$, or $R^2$ with $R^3$, or $R^3$ with $R^4$ together form an alkylenedioxy ring" is methylenedioxy group, ethylenedioxy group or propylenedioxy group.

In the formula (I), the repetition number m is preferably 0 or an integer of 1 to 5, more preferably 0 or an integer of 1 to 3, further preferably 0 or an integer of 1 or 2, and most preferably 0 or 1. The repetition number n is preferably 0 or an integer of 1 to 3, and more preferably 1 or 2.

The "$C_{2-6}$ alkenyl group" in the formula (I) means an alkenyl group having two to six carbon atoms and includes a linear or branched alkenyl group having two to six carbon atoms, such as vinyl group, allyl group, 1-propenyl group, isopropenyl group, 1-buten-1-yl group, 1-buten-2-yl group, 1-buten-3-yl group, 2-buten-1-yl group or 2-buten-2-yl group, of which vinyl group, allyl group and isopropenyl group are preferred.

The "$C_{2-6}$ alkynyl group" in the formula (I) means an alkynyl group derived from an alkyne having two to six carbon atoms. A suitable group is a linear or branched alkynyl group having two to six carbon atoms, such as ethynyl group, 1-propynyl group, 2-propynyl group, butynyl group, pentynyl group or hexynyl group.

The "2,2-(alkylenedioxy)ethyl group" in the formula (I) means a group (acetal group) corresponding to ethyl group except with a cyclic alkylenedioxy group replacing terminal carbon atoms thereof. A preferred group is 2,2-(ethylenedioxy)ethyl group (also called as (1,3-dioxolan-2-yl)methyl group), 2,2-(propylenedioxy)ethyl group (also called as (1,3-dioxan-2-yl)methyl group) or 2,2-(butylenedioxy)ethyl group (also called as (1,3-dioxepan-2-yl)methyl group), of which 2,2-(ethylenedioxy)ethyl group is more preferred.

The "heterocyclic ring" in the formula (I) means a ring containing one to four hetero atoms such as nitrogen atom, sulfur atom or oxygen atom and includes an "5 to 14-membered aromatic heterocyclic ring" and a "5 to 10-membered non-aromatic heterocyclic ring". A preferred ring in the "heterocyclic ring" includes an aromatic heterocyclic ring such as pyrrole, pyridine, pyridazine, pyrimidine, pyrazine, pyrazole, imidazole, indole, isoindole, indolizine, purine, indazole, quinoline, isoquinoline, quinolizine, phthalazine, naphthyridine, quinoxaline, quinazoline, cinnoline, pteridine, imidazotriazine, pyrazinopyridazine, acridine, phenanthridine, carbazole, carbazoline, perimidine, phenanthroline, phenacine, thiophene, benzothiophene, furan, pyran, cyclopentapyran, benzofuran, isobenzofuran, thiazole, isothiazole, benzthiazole, benzthiadiazole, phenothiazine, isoxazole, furazane, phenoxazine, pyrazoloxazole, imidazothiazole, thienofuran, furopyrrole or pyridoxazine ring, or a non-aromatic heterocyclic ring such as pyrrolidine, pyrroline, piperidine, piperazine, imidazoline, pyrazolidine, imidazolidine, morpholine, tetrahydropyran, aziridine, oxirane, oxathiolane, phthalimide or succinimide ring. Among them, pyridine, pyridazine, pyrimidine, pyrazine, piperidine, piperazine and morpholine ring are more preferred.

The most preferred ring as the ring C in the formula (I) is benzene, pyridine, pyridazine, pyrimidine, pyrazine, piperidine, piperazine, morpholine, cyclopropane, cyclobutane, cyclopentane, cyclohexane, cycloheptane or cyclooctane ring.

The "$C_{1-6}$ alkoxy-alkoxy group" in the formula (I) means a group having the same meaning as the $C_{1-6}$ alkoxy group in the above definition to which another "$C_{1-6}$ alkoxy group" is combined and includes, for example, methoxymethoxy group, methoxyethoxy group, methoxypropoxy group, ethoxymethoxy group, ethoxyethoxy group, ethoxypropoxy group or propoxypropoxy group.

The "aryl group" in the "aryloxy group" in the formula (I) means a cyclic hydrocarbon group constituting an aromatic ring and includes, for example, a monocyclic, bicyclic or tricyclic aryl group such as phenyl group, indenyl group, naphthyl group, azulenyl group, heptalenyl group, anthryl group or phenanthrenyl group. The "aryloxy group" is preferably phenoxy group or naphthyloxy group.

The "aralkyloxy group" in the formula (I) means a group comprising oxygen atom combined with an arylalkyl group, which arylalkyl group comprises an alkyl group having one to six carbon atoms combined with a group having

the same meaning as the aryl group. A preferred aralkyloxy group is, for example, benzyloxy group, phenylethoxy group, phenylpropoxy group or naphthylmethoxy group.

The "substituent" in the description of the "which may be substituted" herein includes, for example, a halogen atom, hydroxyl group, nitrile group, an alkyl group having one to six carbon atoms, a cycloalkyl group having three to eight carbon atoms, an alkoxy group having one to six carbon atoms, an $C_{1-6}$ alkoxy-alkoxy group, an aryloxy group, an aralkyloxy group, a halogenoalkyl group having one to six carbon atoms, a hydroxyalkyl group having one to six carbon atoms, a cyano-$C_{1-6}$ alkyl group, a halogenoalkoxy group having one to six carbon atoms, a hydroxyalkoxy group having one to six carbon atoms, a cyano-$C_{1-6}$ alkoxy group, an acyl group having one to six carbon atoms, nitro group, an amino group which may be substituted, an amide group which may be substituted, mercapto group or a thioalkoxy group having one to six carbon atoms, of which a halogen atom, hydroxyl group and nitrile group are preferred.

A preferred indanone derivative, a pharmacologically acceptable salt thereof or a hydrate of them to be contained in the sigma receptor binding agent relating to the present invention is an indanone derivative selected from:

(1) 1-benzyl-4-[(5,6-dimethoxy-1-indanon)-2-ylidene]methylpiperidine,
(2) 1-benzyl-4-[(5,6-diethoxy-1-indanon)-2-ylidene]methylpiperidine,
(3) 1-benzyl-4-[(1-indanon)-2-yl]methylpiperidine,
(4) 1-benzyl-4-[(5-methoxy-1-indanon)-2-yl]methylpiperidine,
(5) 1-benzyl-4-[(5-ethoxy-6-methoxy-1-indanon)-2-yl]methylpiperidine,
(6) 1-benzyl-4-[(5,6-diethoxy-1-indanon)-2-yl]methylpiperidine,
(7) 1-benzyl-4-[[5,6-di(1-propyloxy)-1-indanon)-2-yl]methylpiperidine,
(8) 1-benzyl-4-[2-[(5,6-dimethoxy-1-indanon)-2-yl]ethyl]piperidine,
(9) 1-benzyl-4-[3-[(5,6-dimethoxy-1-indanon)-2-yl]propyl]piperidine,
(10) 1-(3-fluorobenzyl)-4-[(5,6-dimethoxy-1-indanon)-2-yl]methylpiperidine,
(11) 1-(3-methylbenzyl)-4-[(5,6-dimethoxy-1-indanon)-2-yl]methylpiperidine,
(12) 1-cyclohexylmethyl-4-[(5,6-dimethoxy-1-indanon)-2-yl]methylpiperidine,
(13) 1-benzyl-4-[(2-fluoro-1-indanon)-2-yl]methylpiperidine,
(14) 1-benzyl-4-[(5-methoxy-2-fluoro-1-indanon)-2-yl]methylpiperidine,
(15) 1-benzyl-4-[(5,6-dimethoxy-2-fluoro-1-indanon)-2-yl]methylpiperidine,
(16) 1-benzyl-4-[(5,6-diethoxy-2-fluoro-1-indanon)-2-yl]methylpiperidine,
(17) 1-benzyl-4-[[5,6-di(1-propyloxy)-2-fluoro-1-indanon]-2-yl]methylpiperidine,
(18) 1-benzyl-4-[(5,6-dimethoxy-2-fluoro-1-indanon)-2-yl]piperidine,
(19) 1-benzyl-4-[2-[(5,6-dimethoxy-2-fluoro-1-indanon)-2-yl]ethyl]piperidine,
(20) 1-benzyl-4-[3-[(5,6-dimethoxy-2-fluoro-1-indanon)-2-yl]propyl]piperidine,
(21) 1-(2-fluorobenzyl)-4-[(5,6-dimethoxy-2-fluoro-1-indanon)-2-yl]methylpiperidine,
(22) 1-(3-fluorobenzyl)-4-[(5,6-dimethoxy-2-fluoro-1-indanon)-2-yl]methylpiperidine,
(23) 1-(4-fluorobenzyl)-4-[(5,6-dimethoxy-2-fluoro-1-indanon)-2-yl]methylpiperidine,
(24) 1-(3-methylbenzyl)-4-[(5,6-dimethoxy-2-fluoro-1-indanon)-2-yl]methylpiperidine,
(25) 1-cyclohexylmethyl-4-[(5,6-dimethoxy-2-fluoro-1-indanon)-2-yl]methylpiperidine,
(26) 1-benzyl-4-[(5,6-dimethoxy-2-chloro-1-indanon)-2-yl]methylpiperidine,
(27) 1-benzyl-4-[(5,6-diethoxy-2-chloro-1-indanon)-2-yl]methylpiperidine,
(28) 1-benzyl-4-[(5-ethoxy-6-methoxy-2-chloro-1-indanon)-2-yl]methylpiperidine,
(29) 1-benzyl-4-[(5,6-dimethoxy-2-bromo-1-indanon)-2-yl]methylpiperidine, and
(30) 1-benzyl-4-[(5,6-dimethoxy-2-methyl-1-indanon)-2-yl]methylpiperidine, a pharmacologically acceptable salt thereof or a hydrate of them. It should be noted that the present invention is not limited to these compounds.

The indanone derivative, a pharmacologically acceptable salt thereof or a hydrate of them according to the present invention and/or the indanone derivative, a pharmacologically acceptable salt thereof or a hydrate of them to be contained in the sigma receptor binding agent according to the present invention is not specifically limited, but a preferred example is an indanone derivative selected from:

(1) 1-benzyl-4-[[5,6-(1,2-ethylenedioxy)-1-indanon]-2-ylidene]methylpiperidine,
(2) 1-benzyl-4-[(5-cyclohexyl-1-indanon)-2-ylidene]methylpiperidine,
(3) 1-benzyl-4-[(5-cyclohexyloxy-6-methoxy-1-indanon)-2-ylidene]methylpiperidine,
(4) 1-benzyl-4-[[5-methoxy-6-(2-propyloxy)-1-indanon]-2-ylidene]methylpiperidine,
(5) 1-benzyl-4-[[5,6-(1,2-ethylenedioxy)-1-indanon]-2-yl]methylpiperidine,
(6) 1-benzyl-4-[(5-cyclohexyl-1-indanon)-2-yl]methylpiperidine,
(7) 1-benzyl-4-[(5-cyclohexyloxy-6-methoxy-1-indanon)-2-yl]methylpiperidine,

(8) 1-benzyl-4-[[5-methoxy-6-(2-propyloxy)-1-indanon]-2-yl]methylpiperidine,
(9) 1-benzyl-4-[(6-ethoxy-5-methoxy-1-indanon)-2-yl]methylpiperidine,
(10) 1-benzyl-4-[[6-methoxy-5-(1-propyloxy)-1-indanon]-2-yl]methylpiperidine,
(11) 1-benzyl-4-[(5-cyanomethoxy-6-methoxy-1-indanon)-2-yl]methylpiperidine,
(12) 1-cyclopentylmethyl-4-[(5,6-diethoxy-1-indanon)-2-yl]methylpiperidine,
(13) 1-cyclohexylmethyl-4-[(5,6-diethoxy-1-indanon)-2-yl]methylpiperidine,
(14) 1-cycloheptylmethyl-4-[(5,6-diethoxy-1-indanon)-2-yl]methylpiperidine,
(15) 1-cyclooctylmethyl-4-[(5,6-diethoxy-1-indanon)-2-yl]methylpiperidine,
(16) 1-(2-fluorobenzyl)-4-[(5,6-diethoxy-1-indanon)-2-yl]methylpiperidine,
(17) 1-(3-fluorobenzyl)-4-[(5,6-diethoxy-1-indanon)-2-yl]methylpiperidine,
(18) 1-(4-fluorobenzyl)-4-[(5,6-diethoxy-1-indanon)-2-yl]methylpiperidine,
(19) 1-(2-chlorobenzyl)-4-[(5,6-diethoxy-1-indanon)-2-yl]methylpiperidine,
(20) 1-(3-chlorobenzyl)-4-[(5,6-diethoxy-1-indanon)-2-yl]methylpiperidine,
(21) 1-(4-chlorobenzyl)-4-[(5,6-diethoxy-1-indanon)-2-yl]methylpiperidine,
(22) 1-(2-methylbenzyl)-4-[(5,6-diethoxy-1-indanon)-2-yl]methylpiperidine,
(23) 1-(3-methylbenzyl)-4-[(5,6-diethoxy-1-indanon)-2-yl]methylpiperidine,
(24) 1-(4-methylbenzyl)-4-[(5,6-diethoxy-1-indanon)-2-yl]methylpiperidine,
(25) 1-benzyl-4-[(6-ethoxy-5-methoxy-2-fluoro-1-indanon)-2-yl]methylpiperidine,
(26) 1-benzyl-4-[(5-ethoxy-6-methoxy-2-fluoro-1-indanon)-2-yl]methylpiperidine,
(27) 1-benzyl-4-[[6-methoxy-5-(1-propyloxy)-2-fluoro-1-indanon]-2-yl]methylpiperidine,
(28) 1-(2-fluorobenzyl)-4-[(5,6-diethoxy-2-fluoro-1-indanon)-2-yl]methylpiperidine,
(29) 1-(3-fluorobenzyl)-4-[(5,6-diethoxy-2-fluoro-1-indanon)-2-yl]methylpiperidine,
(30) 1-(4-fluorobenzyl)-4-[(5,6-diethoxy-2-fluoro-1-indanon)-2-yl]methylpiperidine,
(31) 1-(2-chlorobenzyl)-4-[(5,6-diethoxy-2-fluoro-1-indanon)-2-yl]methylpiperidine,
(32) 1-(3-chlorobenzyl)-4-[(5,6-diethoxy-2-fluoro-1-indanon)-2-yl]methylpiperidine,
(33) 1-(4-chlorobenzyl)-4-[(5,6-diethoxy-2-fluoro-1-indanon)-2-yl]methylpiperidine,
(34) 1-(2-methylbenzyl)-4-[(5,6-diethoxy-2-fluoro-1-indanon)-2-yl]methylpiperidine,
(35) 1-(3-methylbenzyl)-4-[(5,6-diethoxy-2-fluoro-1-indanon)-2-yl]methylpiperidine,
(36) 1-(4-methylbenzyl)-4-[(5,6-diethoxy-2-fluoro-1-indanon)-2-yl]methylpiperidine,
(37) 1-cyclopentylmethyl-4-[(5,6-diethoxy-2-fluoro-1-indanon)-2-yl]methylpiperidine,
(38) 1-cyclohexylmethyl-4-[(5,6-diethoxy-2-fluoro-1-indanon)-2-yl]methylpiperidine,
(39) 1-cycloheptylmethyl-4-[(5,6-diethoxy-2-fluoro-1-indanon)-2-yl]methylpiperidine,
(40) 1-cyclooctylmethyl-4-[(5,6-diethoxy-2-fluoro-1-indanon)-2-yl]methylpiperidine,
(41) 1-benzyl-4-[(5-cyanomethoxy-6-methoxy-2-fluoro-1-indanon)-2-yl]methylpiperidine,
(42) 1-(3,4-difluorobenzyl)-4-[(5,6-diethoxy-1-indanon)-2-yl]methylpiperidine,
(43) 1-(3,5-difluorobenzyl)-4-[(5,6-diethoxy-1-indanon)-2-yl]methylpiperidine,
(44) 1-(3,4-difluorobenzyl)-4-[(5,6-diethoxy-2-fluoro-1-indanon)-2-yl]methylpiperidine, and
(45) 1-(3,5-difluorobenzyl)-4-[(5,6-diethoxy-2-fluoro-1-indanon)-2-yl]methylpiperidine, a pharmacologically acceptable salt thereof or a hydrate of them.

In the description of the present invention, there is the case where the structural formula of a compound represents a definite isomer. However, the present invention includes all isomers such as geometrical isomers, optical isomers based on asymmetric carbon, stereoisomers and tautomers, and mixtures thereof, is not limited by the description of the formula illustrated for the sake of convenience and can be any one isomer or a mixture thereof. Accordingly, although it is possible that an asymmetric carbon atom is present in a molecule and accordingly that optically active substance and racemic substance may be present, the present invention is not limited thereto but covers any of them. Further, crystal polymorphism may be present but, again, there is not limitation but any of single crystal form or a mixture will do. The compound or its salt related to the present invention may be an anhydride or a hydrate.
The "pharmacologically acceptable salt" in the present description is not specifically limited, as long as it can form a pharmacologically acceptable salt with the compound contained in the sigma receptor binding agent according to the present invention, but preferred examples are a hydrohalide salt, such as hydrofluoride, hydrochloride, hydrobromide or hydroiodide; a salt of an inorganic acid, such as sulfate, nitrate, perchlorate, phosphate, carbonate or hydrogen carbonate; an organic carboxylate, such as acetate, oxalate, maleate, tartrate, fumarate or citrate; a salt of an organic sulfonic acid, such as methanesulfonate, trifluoromethanesulfonate, ethanesulfonate, benzenesulfonate, toluenesulfonate or camphorsulfonate; a salt of an amino acid, such as aspartate or glutamate; a quaternary amine salt; a salt of an alkali metal, such as sodium salt or potassium salt; or a salt of an alkaline earth metal, such as magnesium salt or calcium salt. The "pharmacologically acceptable salt" is more preferably hydrochloride or oxalate.
The indanone derivative, a pharmacologically acceptable salt thereof or a hydrate of them relating to the

present invention and/or the indanone derivative, a pharmacologically acceptable salt thereof or a hydrate of them to be contained in the sigma receptor binding agent relating to the present invention can be easily prepared according to a production method, or from a compound, described in, for example, JP-A 2-169569, 2001-139547 and 2000-319258 by the present inventors.

When the compound contained as an active ingredient in the sigma receptor binding agent relating to the present invention is obtained as a free form, it can be converted into a salt according to a conventional procedure. An isomer of the compound can be purified and isolated according to a conventional separation means such as recrystallization or chromatography. If an optically active isomer is required, it can be obtained, for example, by asymmetrically reducing an enone, optical resolving a racemate, or using an optically active reagent such as a fluorinating agent.

The indanone derivative, a pharmacologically acceptable salt thereof or a hydrate of them relating to the present invention and/or the indanone derivative, a pharmacologically acceptable salt thereof or a hydrate of them to be contained in the sigma receptor binding agent relating to the present invention has a very high sigma receptor binding action and also has a very excellent acetylcholinesterase inhibitory action.

The sigma receptor binding agent according to the present invention can be formulated into a pharmaceutical preparation according to a conventional procedure. Preferred dosage forms are tablets, coated tablets such as film-coated tablets or sugar-coated tablets, fine granules, granules, powders, capsules, syrups, troches, inhalants, suppositories, injections, ointments, eye drops, nasal drops, ear drops, cataplasms, and lotions. In the formulation, generally used fillers, disintegrators, binders, lubricants, coloring agents and flavoring agents, and if necessary, stabilizers, emulsifiers, absorption promoters, surfactants, pH adjusting agents, antiseptics, and antioxidants can be used. They can be formulated according to a conventional procedure using components generally used as raw materials for pharmaceutical preparations. Examples of such components include animal and vegetable oils such as soybean oil, beef tallow and synthetic glycerides; hydrocarbons such as liquid paraffins, squalane and solid paraffins; ester oils such as octyldodecyl myristate and isopropyl myristate; higher alcohols such as cetostearyl alcohol and behenyl alcohol; silicone resins; silicone oils; surfactants such as polyoxyethylene fatty acid esters, sorbitan fatty acid esters, glycerin fatty acid esters, polyoxyethylene sorbitan fatty acid esters, polyoxyethylene hydrogenated castor oils and polyoxyethylene-polyoxypropylene block copolymers; water-soluble polymers such as hydroxyethyl cellulose, poly(acrylic acid)s, carboxyvinyl polymers, polyethylene glycol, polyvinylpyrrolidone and methylcellulose; lower alcohols such as ethanol and isopropanol; polyhydric alcohols such as glycerol, propylene glycol, dipropylene glycol and sorbitol; sugars such as glucose and sucrose; inorganic powders such as silicic anhydride, magnesium aluminium silicate and aluminium silicate; and purified water. The fillers include, for example, lactose, corn starch, sucrose, glucose, mannitol, sorbitol, crystalline cellulose and silicon dioxide; the binders include, for example, polyvinyl alcohol, polyvinyl ether, methylcellulose, ethylcellulose, gum arabic, gum tragacanth, gelatin, shellac, hydroxypropylmethyl cellulose, hydroxypropyl cellulose, polyvinylpyrrolidone, polypropylene glycol-polyoxyethylene block polymers and meglumine; the disintegrators include, for example, starch, agar, gelatin powder, crystalline cellulose, calcium carbonate, sodium hydrogen carbonate, calcium citrate, dextrin, pectin and carboxymethylcellulose calcium; the lubricants include, for example, magnesium stearate, talc, polyethylene glycol, silica, and hardened vegetable oils; the coloring agents can be any coloring agents which are approved to add to pharmaceutical preparations; the flavoring agents include, for example, cocoa powder, menthol, aromatic powder, peppermint oil, borneol and cinnamon powder. It should be noted that the present invention is not limited to these additives.

The oral preparation is produced by mixing the compound, a salt thereof or a hydrate of them as an active ingredient with a filler, and if necessary, a binder, disintegrator, lubricant, coloring agent, flavoring agent, and other components, and formulating the mixture according to a conventional procedure into, for example, a powder, fine granules, granules, tablet, coated tablet or capsule. The tablets and granules can be appropriately coated with, for example, a sugar according to necessity. The syrups or injection preparations can be prepared according to a conventional procedure by adding a pH adjusting agent, solubilizer, and isotonizing agent, and if necessary, a solubilizing agent, stabilizer, and other components. The external preparations can be produced according to a conventional procedure not specifically limited. Base materials for use herein can be any raw materials generally used in, for example, pharmaceutical preparations (medicaments), quasi drugs and cosmetics. Such raw materials include, for example, animal and vegetable oils, mineral oils, ester oils, waxes, higher alcohols, fatty acids, silicone oils, surfactants, phospholipids, alcohols, polyhydric alcohols, water-soluble polymers, clay minerals, and purified water. Where necessary, any of pH adjusting agents, antioxidants, chelating agents, antiseptics and antimolds, coloring agents, flavors and others can be added. In addition, components having differentiation-inducing action, blood-flow accelerators, bactericides, anti-inflammatory agents, cell activators, vitamins, amino acids, humectants, keratolytic agents, and other components can be added according to necessity. The dose of the sigma receptor binding agent according to the present invention varies depending on the degree of symptom, age, sex, body weight, administration mode, type of the salt, concrete type of the disease and other factors. Generally, the agent may be administered to an adult in one to several divided doses at a daily dose of about 30 μg to about 10 g, preferably 100 μg to 5 g, and more preferably 100 μg to 100 mg for oral administration, or about 30 μg to about 1 g, preferably 100 μg to 500 mg, and more preferably 100 μg

to 30 mg for injection administration.

The following sigma receptor binding assay as an example of the advantages of the present invention was performed to demonstrate the efficacy of the indanone derivative, a pharmacologically acceptable salt thereof or a hydrate of them relating to the present invention and/or the indanone derivative, a pharmacologically acceptable salt thereof or a hydrate of them to be contained in the sigma receptor binding agent relating to the present invention. These compounds also have an acetylcholinesterase inhibitory action, and an example of their efficacy is also shown below. It should be noted that the use of the compounds relating to the present invention is not limited to them.

Sigma Receptor Binding Assay

(1) Preparation of Receptor

The receptor was prepared by the following procedure according to the method of Weber et al. (Proc. Natl. Acad. Sci. 83, 8784-8788, 1986). Ten male guinea pigs (Crj, Hartley, from Charles River Japan, Inc.) (body weight: 234 g to 260 g) were decapitated, exsanguinated and died. The brain was immediately removed, homogenized with ten volumes of 50 mM Tris-HCl (pH 7.4) using a Teflon homogenizer and was centrifuged at a rate of 50000xg at 4°C for 20 minutes. The washing procedure was repeated once more under the same conditions, and the resulting crude membrane fraction was subjected to the assay.

(2) Assay Method

An assay was performed using the indanone derivatives shown in Examples 1, 13, 29, 22, 27, 28, 30 and 33 below as a test compound and donepezil hydrochloride (1-benzyl-4-[(5,6-dimethoxy-1-indanon)-2-yl]methylpiperidine hydrochloride) as a control.

In an assay tube (Daiichi Tube, from DRL) were incubated 0.1 mL of a solution of the test compound, 0.3 mL of a 50 mM Tris-HCl buffer (pH 7.4), 0.1 mL of $^3$H-DTG solution (3.2 nM) and 0.5 mL of the receptor (guinea pig brain crude membrane fraction, 5 mg tissue) at 25°C for 1 hour. After incubation, the mixture was filtrated using a glass filter (GF/B, from Whatman) which had been treated with 0.3% ethyleneimine. The filter paper was transferred to a vial for radioactivity measurement, mixed with 5 mL of a liquid scintillation cocktail (Atomlight, from PACKARD), and the radioactivity on the filter was determined by a liquid scintillation counter (Model 1500, from PACKARD) for 2 minutes. The specific binding and the non-specific binding in the absence of the test compound were determined in the same way as above except for using 50% DMSO solution instead of the test compound and using a 100 µM Haloperidol solution instead of the test compound, respectively.

(3) Determination of Inhibition

The inhibitions of the test compound at individual concentrations were determined from the ratio of the specific binding in the presence of the test compound to the specific binding in the absence thereof. The dose-response curve was regressed according to a logit-log model in which the ratio of the specific binding in the presence of the test compound to the specific binding in the absence thereof was subjected to logit transformation and plotted against the common logarithm value of each concentration. The $IC_{50}$, i.e., the concentration of the test compound inhibits $^3$H-DTG binding to the guinea pig brain crude membrane fraction by 50% was determined by calculation according to the above-obtained regression equation.

The sigma receptor inhibitions ($IC_{50}$) of the compounds relating to the present invention are as follows.

Table 1

| Test compound | Sigma receptor inhibition $IC_{50}$ (nM) |
|---|---|
| Example 1 | 5.1 |
| Example 13 | 1.6 |
| Example 19 | 5.0 |
| Example 22 | 1.3 |
| Example 27 | 1.7 |
| Example 28 | 2.3 |
| Example 30 | 3.7 |
| Example 33 | 7.6 |

Table 1 (continued)

| Test compound | Sigma receptor inhibition $IC_{50}$ (nM) |
|---|---|
| *) Control | 18.7 |

*) Control: Donepezil hydrochloride

Table 2

| Test compound | Sigma receptor inhibition $IC_{50}$ (nM) |
|---|---|
| Example 37 | 4.3 |
| Example 41 | 3.0 |
| Example 43 | 1.5 |
| Example 48 | 2.8 |
| Example 51 | 1.1 |
| Example 52 | 1.1 |
| Example 58 | 1.3 |
| Example 68 | 2.6 |
| *) Control | 18.7 |

*) Control: Donepezil hydrochloride

The present invention can provide novel sigma receptor binding agents. The above results clearly show that the indanone derivatives, pharmacologically acceptable salts thereof or hydrates of them according to the present invention, and/or the indanone derivatives pharmacologically acceptable salts thereof or hydrates of them to be contained in the sigma receptor binding agents according to the present invention are useful as agents for treating or preventing a disease in which a sigma receptor is involved and are useful as agents for treating or preventing a disease against which a sigma receptor antagonistic action or sigma receptor agonistic action is efficacious.

Thus, the sigma receptor binding agents according to the present invention are useful not only as antipsychotic agents but also as agents for treating or preventing a schizophrenia, depression and anxiety and agents for improving intellectual function. Further, the sigma receptor binding agents relating to the present invention are very satisfactory in adverse drug action, number of administration per certain period, and administration mode. Inhibitory effect on acetylcholinesterase

Using a rat brain homogenate as a source of acetylcholinesterase, the esterase activity was determined in accordance with the method of Ellman et al (Ellman. G.L., Courtney, K.D., Andres, V. and Featherstone, R.M., Biochem. Pharmacol., 7, 88 to 95, 1961). To DTNB (5,5'-dithiobis(2-nitrobenzoic acid)), acethylthiocholine (as a substrate) and a test compound was added the rat brain homogenate and incubated. Then, the resulting yellow product produced by the reaction of the resulting thiocholine with DTNB was determined for the change in absorbance at 412 nm, to determine the acethylcholinesterase activity. The acetylcholinesterase inhibitory action of each test compound was determined in terms of 50% inhibitory concentration ($IC_{50}$).

As the test compounds, indanone derivatives according to Examples 1, 2, 7, 9 and 15 were used after dissolved in distilled water or ethanol. The inhibitory action of donepezil hydrochloride (1-benzyl-4-[(5,6-dimethoxy-1-indanon)-2-yl]methylpiperidine hydrochloride) as a control was determined in the same way as above.

The acetylcholinesterase inhibitions ($IC_{50}$) of the compounds relating to the present invention are shown below.

Table 3

| Test compound | Acetylcholinesterase inhibition $IC_{50}$ (nM) |
|---|---|
| Example 1 | 7.2 |
| Example 2 | 2.7 |
| Example 7 | 2.4 |
| Example 9 | 3.4 |
| Example 15 | 9.1 |
| *) Control | 3.9 |

*) Control: Donepezil hydrochloride

Table 4

| Test compound | Acetylcholinesterase inhibition $IC_{50}$ (nM) |
|---|---|
| Example 37 | 0.73 |
| Example 38 | 0.28 |
| Example 44 | 0.75 |
| Example 47 | 1.0 |
| Example 50 | 1.9 |
| *) Control | 3.9 |

*) Control: Donepezil hydrochloride

These results clearly show that the indanone derivatives, pharmacologically acceptable salts thereof or hydrates of them according to the present invention, and/or the indanone derivatives, pharmacologically acceptable salts thereof or hydrates of them to be contained in the sigma receptor binding agents according to the present invention have an excellent acetylcholinesterase inhibitory action and are useful as agents for treating or preventing a disease against which an acetylcholinesterase inhibitory action is efficacious. Namely, they are also useful as agents for preventing, treating or improving Alzheimer-type dementia and other senile dementia, cerebrovascular dementia, attention-deficit hyperactive disorder, glaucoma, myasthenia gravis and/or migraine.

Examples

The present invention will be illustrated in further detail with reference to the following Examples, which are not intended to limit the scope of the invention.

Example 1: Synthesis of 1-benzyl-4-[(6-ethoxy-5-methoxy-1-indanon)-2-yl]methylpiperidine hydrochloride

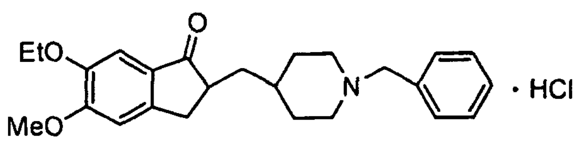

0.20 g of 1-benzyl-[(6-hydroxy-5-methoxy-1-indanon)-2-yl]methylpiperidine described in JP-A 2-169569 was dissolved in 20 ml of tetrahydrofuran (THF), followed by addition of 0.064 ml of ethanol, 0.29 g of triphenylphosphine and 0.17 ml of diethyl azodicarboxylate. After stirring at room temperature overnight, the mixture was evaporated. To the residue was added water, and the mixture was extracted with ethyl acetate. The organic layer was washed with brine, dried over magnesium sulfate ($MgSO_4$) and evaporated. The resulting residue was purified by silica gel column chromatography (NH-silica gel; n-hexane/ethyl acetate system), to give 0.18 g (83%) of the free form of the title compound as a pale yellow oil.

$^1$H-NMR (400Mz:$CDCl_3$)δ : 1.24-1.42 (4H,m), 1.48 (3H,t,*J*=6.8Hz), 1.63-1.77 (2H,m), 1.88-2.01 (3H,m), 2.66-2.73 (2H, m), 2.86-2.94 (2H,m), 3.22 (1H,dd,*J*=8Hz,*J*=17.6Hz), 3.51 (2H,s), 3.95 (3H,s), 4.12 (2H,q,*J*=6.8Hz), 6.85 (1H,s), 7.16 (1H,s), 7.23-7.34 (5H,m).

The product was converted into a hydrochloride in a conventional manner and recrystallized from ethanol/t-butyl methyl ether, to give the title compound as pale yellowish white crystals.

ESI-MS : m/z = 394 ($M+H^+$).

Example 2: Synthesis of 1-benzyl-4-[[5-methoxy-6-(1-propyloxy)-1-indanon]-2-yl]methylpiperidine hydrochloride

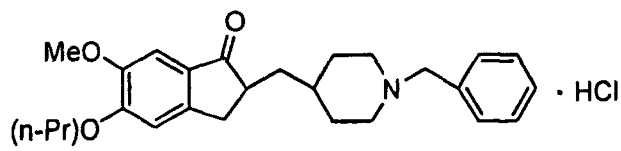

The free form of the title compound was obtained as a pale yellow oil in the same way as Example 1 except for using 1-benzyl-[(5-hydroxy-6-methoxy-1-indanon)-2-yl]methylpiperidine described in JP-A 2-169569 and 1-propanol (yield; 83%).
$^1$H-NMR (400Mz:$CDCl_3$)δ : 1.06 (3H,t, *J*=6.8Hz), 1.26-1.54 (4H,m), 1.63-1.76 (2H,m), 1.86-2.01 (5H,m), 2.64-2.73 (2H, m), 2.86-2.94 (2H,m), 3.21 (1H,dd,*J*=8Hz,*J*=17.6Hz), 3.50 (2H,s), 3.88 (3H,s), 4.05 (2H,t,*J*=6.8Hz), 6.83 (1H,s), 7.16 (1H,s), 7.22-7.33 (5H,m).
The product was converted into a hydrochloride in a conventional manner and recrystallized from ethanol/t-butyl methyl ether, to give the title compound as pale yellowish white crystals.
ESI-MS : m/z = 408 ($M+H^+$).

Example 3: Synthesis of 1-cyclopentylmethyl-4-[(5,6-diethoxy-1-indanon)-2-yl]methylpiperidine hydrochloride

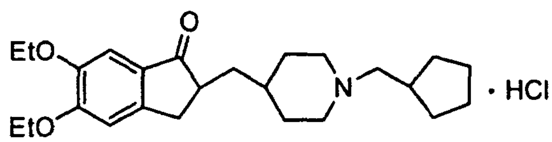

3-1) 3-(3,4-Diethoxy)propionic acid

52.3 g of 3-(3,4-dihydroxy)propionic acid was dissolved in 500 ml of ethanol, followed by addition of 5 ml of concentrated sulfuric acid. After heating under reflux for 3 hours, the mixture was left stand to cool to room temperature and evaporated. The resulting residue was extracted with a saturated aqueous solution of sodium bicarbonate and ethyl acetate. The organic layer was washed with brine, dried over magnesium sulfate ($MgSO_4$), and evaporated.
The resulting residue was dissolved in 320 ml of dimethylformamide (DMF), followed by addition of 103 g of potassium carbonate and 59.7 ml of iodoethane. After stirring at 50°C for 5 hours, the mixture was left stand to cool to room temperature, diluted with water and extracted with ethyl acetate. The organic layer was washed with brine, dried over magnesium sulfate ($MgSO_4$), and evaporated.
The resulting residue was dissolved in 500 ml of THF, followed by addition of 500 ml of a 1 N aqueous solution of sodium hydroxide. After heating under reflux for 1 hour, the mixture was left stand to cool to room temperature, diluted with water and washed with diethyl ether. Further, the aqueous layer was adjusted to pH 2 with concentrated hydrochloric acid, and then the precipitated solid was collected by filtration and dried. The product was recrystallized from ethyl acetate, to give 61.2 g (total yield in the three steps; 90%) of the title compound as white crystals.

3-2) 5,6-Diethoxy-1-indanone

30.0 g of the product was dissolved in 300 ml of toluene, followed by addition of 27.6 ml of thionyl chloride. After heating under reflux for 2 hours, the mixture was left stand to cool to room temperature and then evaporated.
The residue was dissolved in 300 ml of methylene chloride, followed by addition of 20.1 g of aluminum chloride under ice-cooling. After stirring for 2 hours as it was, the mixture was poured onto ice water and extracted with methylene chloride. The organic layer was washed with brine, dried over magnesium sulfate ($MgSO_4$), and evaporated. The residue was recrystallized from ethyl acetate, to give 24.1 g (total yield in the two steps; 87%) of the title compound as white crystals.

3-3) 1-Benzyl-4-[(5,6-diethoxy-1-indanon)-2-ylidene]methylpiperidine

To a solution of 24.1 g of 5,6-diethoxy-1-indanone in 70 ml of THF was added 26.7 g of 1-benzyl-4-formylpiperidine in 100 ml of THF, followed by addition of a solution of 23.2 g of sodium methoxide (28% methanol solution) in 30 ml of THF under ice-cooling. After stirring for 2 hours as it was, the mixture was poured onto ice water, and the precipitated solid was collected by filtration and dried. The product was recrystallized from ethyl acetate-ethanol, to give 39.6 g (89%) of the title compound as white crystals.

3-4) 4-[(5,6-Diethoxy-1-indanon)-2-yl]methylpiperidine

39.4 g of 1-benzyl-4-[(5,6-diethoxy-1-indanon)-2-ylidene]methylpiperidine was dissolved in 500 ml of methanol and 200 ml of THF, 4 g of 20% palladium hydroxide/carbon was added thereto, and the mixture was subjected to hydrogenation at room temperature at normal pressure overnight. After filtering off the catalyst, the filtrate was evaporated, to give 30.8 g (quantitative) of the title compound as a white powder.

3-5) Mesyloxymethylcyclopentane

1.00 g of cyclopentanemethanol was dissolved in 20 ml of THF, followed by addition of 4.17 ml of triethylamine (TEA) and 0.95 ml of mesyl chloride. After stirring at room temperature for 30 minutes, the mixture was diluted with water and extracted with ethyl acetate. The organic layer was washed with brine, dried over magnesium sulfate ($MgSO_4$), and evaporated, to give 1.73 g (97%) of the title compound.

3-6) 1-Cyclopentylmethyl-4-[(5,6-diethoxy-1-indanon)-2-yl]methylpiperidine

To a solution of 0.31 g of mesyloxymethylcyclopentane in 15 ml of DMF were added 0.50 g of 4-[(5,6-diethoxy-1-indanon)-2-yl]methylpiperidine and 0.26 g of potassium carbonate, followed by stirring at 70°C overnight. The mixture was left stand to cool to room temperature, diluted with water and then extracted with ethyl acetate. The organic layer was washed with brine, dried over magnesium sulfate ($MgSO_4$), and evaporated. The resulting residue was purified by silica gel column chromatography (NH-silica gel; n-hexane/ethyl acetate system), to give 0.33 g (68%) of the free form of the title compound as a pale yellow oil.
$^1$H-NMR (400Mz:$CDCl_3$)δ : 1.13-1.23 (2H,m), 1.26-1.43 (3H,m), 1.44-1.82 (9H,m), 1.47 (3H,t,*J*=6.8Hz), 1.51 (3H,t, *J*=6.8Hz), 1.86-1.96 (3H,m), 2.06 (1H,qui,*J*=6.8Hz), 2.27 (2H,d,*J*=6.8Hz), 2.65-2.73 (2H,m), 2.89-2.98 (2H,m), 3.22 (1H,dd,*J*=8Hz,*J*=17.6Hz), 4.11 (2H,q,*J*=6.8Hz), 4.17 (2H,q,*J*=6.8Hz), 6.83 (1H,s), 7.16 (1H,s).
The product was converted into a hydrochloride in a conventional manner and recrystallized from ethanol/t-butyl methyl ether, to give the title compound as pale yellowish white crystals.
ESI-MS : m/z = 400 ($M+H^+$).

Example 4: Synthesis of 1-cyclohexylmethyl-4-[(5,6-diethoxy-1-indanon)-2-yl]methylpiperidine hydrochloride

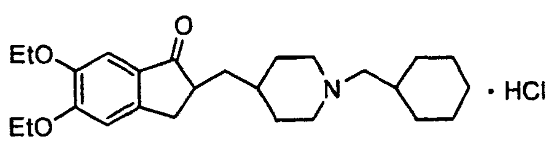

0.50 g of 4-[(5,6-diethoxy-1-indanon)-2-yl]methylpiperidine was dissolved in 10 ml of methylene chloride, followed by addition of 0.21 ml of cyclohexanecarbaldehyde, 0.14 ml of acetic acid and then 0.50 g of sodium triacetoxyborohydride. After stirring at room temperature for 4 hours, a saturated aqueous solution of sodium carbonate was added thereto and the mixture was extracted with ethyl acetate. The organic layer was washed with brine, dried over magnesium sulfate ($MgSO_4$), and evaporated. The resulting residue was purified by silica gel column chromatography (NH-silica gel; n-hexane/ethyl acetate system), to give 0.59 g (91%) of the free form of the title compound as a pale yellow oil.
$^1$H-NMR (400Mz:$CDCl_3$)δ : 0.80-0.94 (2H,m), 1.10-1.95 (18H,m), 1.47 (3H,t,*J*=6.8Hz), 1.51 (3H,t,*J*=6.8Hz), 2.09 (2H, d,*J*=7.2Hz), 2.65-2.73 (2H,m), 2.82-2.90 (2H,m), 3.22 (1H,dd,*J*=8Hz,*J*=17.6Hz), 4.11 (2H,q,*J*=6.8Hz), 4.17 (2H,q, *J*=6.8Hz), 6.83 (1H,s), 7.16 (1H,s).

The product was converted into a hydrochloride in a conventional manner and recrystallized from ethanol/t-butyl methyl ether, to give the title compound as pale yellowish white crystals.
ESI-MS : m/z = 414 (M+H$^+$).

Example 5: Synthesis of 1-cycloheptylmethyl-4-[(5,6-diethoxy-1-indanon)-2-yl]methylpiperidine hydrochloride

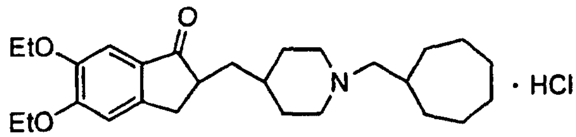

5-1) Methyl cycloheptanecarboxylate

The title compound was obtained in the same way as Example 3-1 except for using cycloheptanecarboxylic acid and methanol (yield; 81%).

5-2) Cyclopentanemethanol

1.76 g of methyl cycloheptanecarboxylate was dissolved in 20 ml of THF, followed by addition of 0.53 g of a 80% lithium aluminum hydride under ice-cooling. After stirring for 1 hour as it was, a 1 N aqueous solution of sodium hydroxide and ethyl acetate were added thereto, and solid matters were filtered off. The filtrate was dried over magnesium sulfate ($MgSO_4$), and then evaporated, to give 0.96 g (66%) of the title compound.

5-3) Mesyloxymethylcycloheptane

The title compound was obtained in the same way as Example 3-5 (yield; 93%).

5-4) 1-Cycloheptylmethyl-4-[(5,6-diethoxy-1-indanon)-2-yl]methylpiperidine

The free form of the title compound was obtained as a pale yellow oil in the same way as Example 3-6 (yield; 74%).
$^1$H-NMR (400Mz:$CDCl_3$)δ : 1.05-1.15 (2H,m), 1.24-1.44 (20H,m), 1.47 (3H,t,*J*=6.8Hz), 1.51 (3H,t,*J*=6.8Hz), 2.07 (2H,d,*J*=7.6Hz), 2.65-2.73 (2H,m), 2.82-2.90 (2H,m), 3.22 (1H,dd,*J*=8Hz,*J*=17.6Hz), 4.11 (2H,q,*J*=6.8Hz), 4.17 (2H,q,*J*=6.8Hz), 6.83 (1H,s), 7.16 (1H,s).

The product was converted into a hydrochloride in a conventional manner and recrystallized from ethanol/t-butyl methyl ether, to give the title compound as pale yellowish white crystals.
ESI-MS : m/z = 428 (M+H$^+$).

Example 6: Synthesis of 1-cyclooctylmethyl-4-[(5,6-diethoxy-1-indanon)-2-yl]methylpiperidine hydrochloride

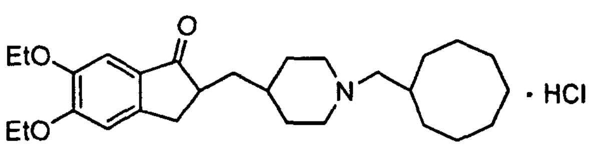

6-1) Mesyloxymethylcyclooctane

The title compound was obtained in the same way as Example 3-5 (yield; 99%).

6-2) 1-Cyclooctylmethyl-4-[(5,6-diethoxy-1-indanon)-2-yl]methylpiperidine

The free form of the title compound was obtained as a pale yellow oil in the same way as Example 3-6 (yield; 86%).
$^1$H-NMR (400Mz:$CDCl_3$)δ : 1.15-1.75 (21H,m), 1.47 (3H,t,*J*=6.8Hz), 1.51 (3H,t,*J*=6.8Hz), 1.80-1.95 (3H,m), 2.05 (2H, d,*J*=7.2Hz), 2.65-2.74 (2H,m), 2.82-2.90 (2H,m), 3.22 (1H,dd,*J*=8.4Hz,*J*=17.6Hz), 4.11 (2H,q,*J*=6.8Hz), 4.17 (2H,q, *J*=6.8Hz), 6.83 (1H,s), 7.16 (1H,s).
The product was converted into a hydrochloride in a conventional manner and recrystallized from ethanol/t-butyl methyl ether, to give the title compound as pale yellowish white crystals.
ESI-MS : m/z = 442 ($M+H^+$).

Example 7: Synthesis of 1-benzyl-4-[(5-cyanomethoxy-6-methoxy-1-indanon)-2-yl]methylpiperidine hydrochloride

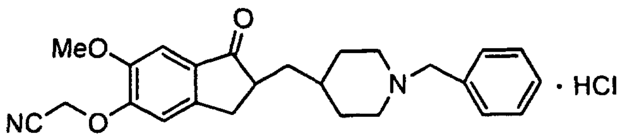

0.25 g of 1-benzyl-[(5-hydroxy-6-methoxy-1-indanon)-2-yl]methylpiperidine was dissolved in 10 ml of DMF, followed by addition of 0.14 g of potassium carbonate and 0.071 ml of acetonitrile bromide. After stirring at 80°C overnight, the mixture was left stand to cool to room temperature, diluted with water and extracted with ethyl acetate. The organic layer was washed with brine, dried over magnesium sulfate ($MgSO_4$), and evaporated. The resulting residue was purified by preparative thin layer silica gel column chromatography (methylene chloride/methanol system), to give 0.12 g (44%) of the free form of the title compound as a pale yellow oil.
$^1$H-NMR (400Mz:$CDCl_3$)δ : 1.30-1.44 (3H,m), 1.44-1.59 (1H,m), 1.65-1.78 (2H,m), 1.86-1.95 (1H,m), 1.98-2.08 (2H, m), 2.68-2.78 (2H,m), 2.90-2.99 (2H,m), 3.22-3.31 (1H,m), 3.56 (2H,s), 3.91 (3H,s), 4.92 (2H,s), 7.04 (1H,s), 7.23-7.35 (5H,m), 7.24 (1H,s).
The product was converted into a hydrochloride in a conventional manner and recrystallized from ethanol/t-butyl methyl ether, to give the title compound as pale yellowish white crystals.
ESI-MS : m/z = 405 ($M+H^+$).

Example 8: Synthesis of 1-(2-fluorobenzyl)-4-[(5,6-diethoxy-1-indanon)-2-yl]methylpiperidine hydrochloride

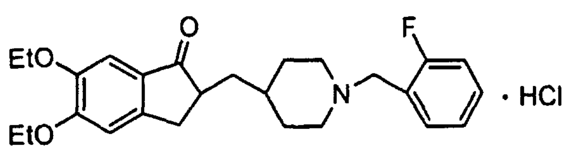

The free form of the title compound was obtained as a pale yellow oil in the same way as Example 4 (yield; 88%).
$^1$H-NMR (400Mz:$CDCl_3$)δ : 1.26-1.45 (4H,m), 1.46 (3H,t,*J*=6.8Hz), 1.51 (3H,t,*J*=6.8Hz), 1.63-1.77 (2H,m), 1.86-1.94 (1H,m), 1.99-2.08 (2H,m), 2.64-2.71 (2H,m), 2.87-2.96 (2H,m), 3.20 (1H,dd,*J*=8Hz,*J*=17.6Hz), 3.59 (2H,s), 4.11 (2H, q,*J*=6.8Hz), 4.16 (2H,q,*J*=6.8Hz), 6.83 (1H,s), 6.99-7.06 (1H,m), 7.11 (1H,dt,*J*=1.2Hz,*J*=7.6Hz), 7.15 (1H,s), 7.20-7.28 (1H,m), 7.38 (1H,dt,*J*=1.6Hz,*J*=7.6Hz).
The product was converted into a hydrochloride in a conventional manner and recrystallized from ethanol/t-butyl methyl ether, to give the title compound as pale yellowish white crystals.
ESI-MS : m/z = 426 ($M+H^+$).

Example 9: Synthesis of 1-(3-fluorobenzyl)-4-[(5,6-diethoxy-1-indanon)-2-yl]methylpiperidine hydrochloride

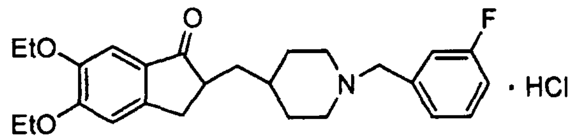

The free form of the title compound was obtained as a pale yellow oil in the same way as Example 4 (yield; 79%).
$^1$H-NMR (400Mz:$CDCl_3$)δ : 1.25-1.44 (4H,m), 1.46 (3H,t,*J*=6.8Hz), 1.51 (3H,t,*J*=6.8Hz), 1.64-1.77 (2H,m), 1.87-2.04 (3H,m), 2.64-2.72 (2H,m), 2.84-2.93 (2H,m), 3.21 (1H,dd,*J*=8Hz,*J*=17.6Hz), 3.50 (2H,s), 4.11 (2H,q,*J*=6.8Hz), 4.16 (2H,q,*J*=6.8Hz), 6.83 (1H,s), 6.90-6.97 (1H,m), 7.04-7.11 (2H,m), 7.15 (1H,s), 7.23-7.30 (1H,m).
The product was converted into a hydrochloride in a conventional manner and recrystallized from ethanol/t-butyl methyl ether, to give the title compound as pale yellowish white crystals.
ESI-MS : m/z = 426 ($M+H^+$).

Example 10: Synthesis of 1-(4-fluorobenzyl)-4-[(5,6-diethoxy-1-indanon)-2-yl]methylpiperidine hydrochloride

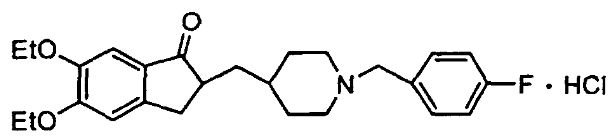

The free form of the title compound was obtained as a pale yellow oil in the same way as Example 4 (yield; 90%).
$^1$H-NMR (400Mz:$CDCl_3$)δ : 1.25-1.40 (4H,m), 1.46 (3H,t,*J*=6.8Hz), 1.51 (3H,t,*J*=6.8Hz), 1.63-1.77 (2H,m), 1.87-2.00 (3H,m), 2.64-2.72 (2H,m), 2.83-2.91 (2H,m), 3.21 (1H,dd,*J*=8Hz,*J*=17.6Hz), 3.46 (2H,s), 4.11 (2H,q,*J*=6.8Hz), 4.16 (2H,q,*J*=6.8Hz), 6.83 (1H,s), 6.96-7.03 (2H,m), 7.15 (1H,s), 7.24-7.30 (2H,m).
The product was converted into a hydrochloride in a conventional manner and recrystallized from ethanol/t-butyl methyl ether, to give the title compound as pale yellowish white crystals.
ESI-MS : m/z = 426 ($M+H^+$).

Example 11: Synthesis of 1-(2-chlorobenzyl)-4-[(5,6-diethoxy-1-indanon)-2-yl]methylpiperidine hydrochloride

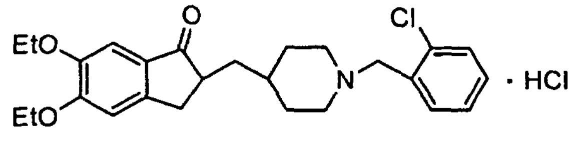

The free form of the title compound was obtained as a pale yellow oil in the same way as Example 4 (yield; 80%).
$^1$H-NMR (400Mz:$CDCl_3$)δ : 1.24-1.45 (4H,m), 1.46 (3H,t,*J*=6.8Hz), 1.51 (3H,t,*J*=6.8Hz), 1.64-1.79 (2H,m), 1.88-1.96 (1H,m), 2.04-2.13 (2H,m), 2.65-2.73 (2H,m), 2.88-2.96 (2H,m), 3.22 (1H,dd,*J*=8Hz,*J*=17.6Hz), 3.61 (2H,s), 4.11 (2H,q,*J*=6.8Hz), 4.17 (2H,q,*J*=6.8Hz), 6.83 (1H,s), 7.14-7.38 (3H,m), 7.16 (1H,s), 7.49 (2H,dd,*J*=1.6Hz,*J*=7.6Hz).
The product was converted into a hydrochloride in a conventional manner and recrystallized from ethanol/t-butyl methyl ether, to give the title compound as pale yellowish white crystals.

ESI-MS : m/z = 442 (M+H+).

Example 12: Synthesis of 1-(3-chlorobenzyl)-4-[(5,6-diethoxy-1-indanon)-2-yl]methylpiperidine hydrochloride

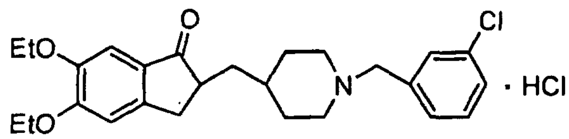

The free form of the title compound was obtained as a pale yellow oil in the same way as Example 4 (yield; 85%).
$^1$H-NMR (400Mz:$CDCl_3$)δ : 1.24-1.42 (4H,m), 1.46 (3H,t,*J*=6.8Hz), 1.51 (3H,t,*J*=6.8Hz), 1.63-1.77 (2H,m), 1.87-2.02 (3H,m), 2.65-2.73 (2H,m), 2.83-2.91 (2H,m), 3.21 (1H,dd,*J*=8.4Hz,*J*=17.6Hz), 3.46 (2H,s), 4.11 (2H,q,*J*=6.8Hz), 4.17 (2H,q,*J*=6.8Hz), 6.83 (1H,s), 7.16 (1H,s), 7.18-7.34 (4H,m).
The product was converted into a hydrochloride in a conventional manner and recrystallized from ethanol/t-butyl methyl ether, to give the title compound as pale yellowish white crystals.
ESI-MS : m/z = 442 (M+H+).

Example 13: Synthesis of 1-(4-chlorobenzyl)-4-[(5,6-diethoxy-1-indanon)-2-yl]methylpiperidine hydrochloride

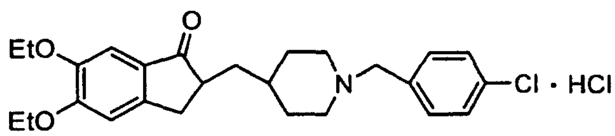

The free form of the title compound was obtained as a pale yellow oil in the same way as Example 4 (yield; 89%).
$^1$H-NMR (400Mz:$CDCl_3$)δ : 1.24-1.40 (4H,m), 1.46 (3H,t,*J*=6.8Hz), 1.51 (3H,t,*J*=6.8Hz), 1.63-1.76 (2H,m), 1.86-2.00 (3H,m), 2.64-2.72 (2H,m), 2.81-2.90 (2H,m), 3.21 (1H,dd,*J*=8Hz,*J*=17.6Hz), 3.45 (2H,s), 4.11 (2H,q,*J*=6.8Hz), 4.16 (2H,q,*J*=6.8Hz), 6.83 (1H,s), 7.15 (1H,s), 7.23-7.34 (4H,m).
The product was converted into a hydrochloride in a conventional manner and recrystallized from ethanol/t-butyl methyl ether, to give the title compound as pale yellowish white crystals.
ESI-MS : m/z = 442 (M+H+).

Example 14: Synthesis of 1-(2-methylbenzyl)-4-[(5,6-diethoxy-1-indanon)-2-yl]methylpiperidine hydrochloride

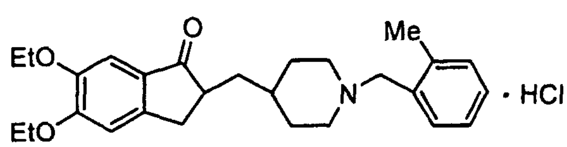

The free form of the title compound was obtained as a pale yellow oil in the same way as Example 4 (yield; 70%).
$^1$H-NMR (400Mz:$CDCl_3$)δ : 1.23-1.39 (4H,m), 1.46 (3H,t,*J*=6.8Hz), 1.51 (3H,t,*J*=6.8Hz), 1.60-1.76 (2H,m), 1.87-2.04 (3H,m), 2.36 (3H,s), 2.65-2.73 (2H,m), 2.84-2.93 (2H,m), 3.21 (1H,dd,*J*=8Hz,*J*=17.6Hz), 3.43 (2H,s), 4.11 (2H,q, *J*=6.8Hz), 4.17 (2H,q,*J*=6.8Hz), 6.83 (1H,s), 7.12-7.30 (4H,m), 7.15 (1H,s).

The product was converted into a hydrochloride in a conventional manner and recrystallized from ethanol/t-butyl methyl ether, to give the title compound as pale yellowish white crystals.
ESI-MS : m/z = 422 (M+H+).

Example 15: Synthesis of 1-(3-methylbenzyl)-4-[(5,6-diethoxy-1-indanon)-2-yl]methylpiperidine hydrochloride

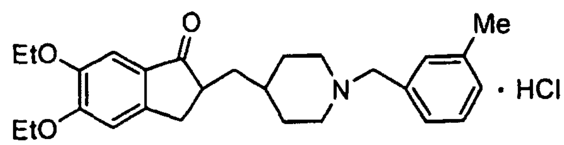

The free form of the title compound was obtained as a pale yellow oil in the same way as Example 4 (yield; 77%).
$^1$H-NMR (400Mz:$CDCl_3$)δ : 1.26-1.43 (4H,m), 1.46 (3H,t,*J*=6.8Hz), 1.51 (3H,t,*J*=6.8Hz), 1.63-1.76 (2H,m), 1.87-2.00 (3H,m), 2.35 (3H,s), 2.64-2.72 (2H,m), 2.86-2.94 (2H,m), 3.21 (1H,dd,*J*=8Hz,*J*=17.6Hz), 3.46 (2H,s), 4.12 (2H,q, *J*=6.8Hz), 4.16 (2H,q,*J*=6.8Hz), 6.83 (1H,s), 7.04-7.15 (3H,m), 7.15 (1H,s), 7.20 (1H,t,*J*=7.6Hz).
The product was converted into a hydrochloride in a conventional manner and recrystallized from ethanol/t-butyl methyl ether, to give the title compound as pale yellowish white crystals.
ESI-MS : m/z = 422 (M+H+).

Example 16: Synthesis of 1-(4-methylbenzyl)-4-[(5,6-diethoxy-1-indanon)-2-yl]methylpiperidine hydrochloride

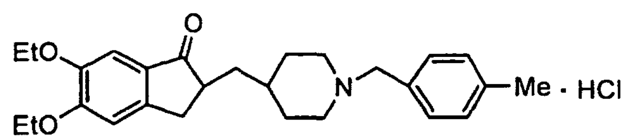

The free form of the title compound was obtained as a pale yellow oil in the same way as Example 4 (yield; 74%).
$^1$H-NMR (400Mz:$CDCl_3$)δ : 1.25-1.42 (4H,m), 1.46 (3H,t,*J*=6.8Hz), 1.51 (3H,t,*J*=6.8Hz), 1.61-1.76 (2H,m), 1.86-2.00 (3H,m), 2.34 (3H,s), 2.64-2.71 (2H,m), 2.85-2.94 (2H,m), 3.20 (1H,dd,*J*=8Hz,*J*=17.6Hz), 3.47 (2H,s), 4.11 (2H,q, *J*=6.8Hz), 4.16 (2H,q,*J*=6.8Hz), 6.83 (1H,s), 7.15 (1H,s), 7.16 (4H,dd,*J*=7.6Hz,*J*=30.8Hz).
The product was converted into a hydrochloride in a conventional manner and recrystallized from ethanol/t-butyl methyl ether, to give the title compound as pale yellowish white crystals.
ESI-MS : m/z = 422 (M+H+).

Example 17: Synthesis of 1-benzyl-4-[[5,6-(1,2-ethylenedioxy)-1-indanon]-2-ylidene]methylpiperidine hydrochloride

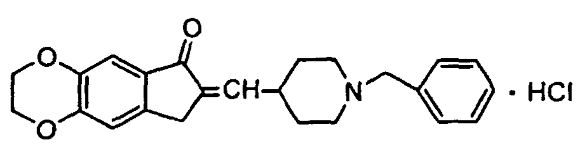

The free form of the title compound was obtained as a pale yellow oil in the same way as Example 3-3 (yield; 67%).

$^1$H-NMR (90Mz:$CDCl_3$)δ : 1.40-2.50 (7H,m), 2.72-3.00 (2H,m), 3.40-3.60 (4H,m), 4.08-4.35 (4H,m), 6.48-6.68 (1H,m), 6.82 (1H,s), 7.10-7.35 (5H,m), 7.20 (1H,s).

The product was converted into a hydrochloride in a conventional manner and recrystallized from methanol/diisopropyl ether, to give the title compound as pale yellowish white crystals.
GC-MS : m/z = 375 ($M^+$).

Example 18: Synthesis of 1-benzyl-4-[(5-cyclohexyl-1-indanon)-2-ylidene]methylpiperidine hydrochloride

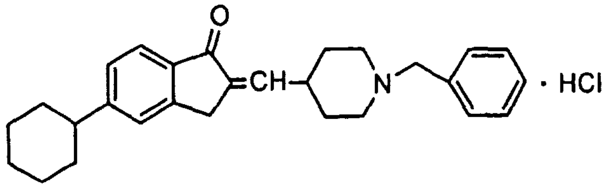

The free form of the title compound was obtained as a pale yellow oil in the same way as Example 3-3 (yield; 22%).
$^1$H-NMR (90Mz:$CDCl_3$)δ : 1.10-3.02 (20H,m), 3.50 (2H,s), 3.50-3.62 (2H,m), 6.55-6.78 (1H,m), 7.02-7.80 (8H,m).

The product was converted into a hydrochloride in a conventional manner and recrystallized from methanol/diisopropyl ether, to give the title compound as pale yellowish white crystals.
GC-MS : m/z = 399 ($M^+$).

Example 19: Synthesis of 1-benzyl-4-[(5-cyclohexyloxy-6-methoxy-1-indanon)-2-ylidene]methylpiperidine hydrochloride

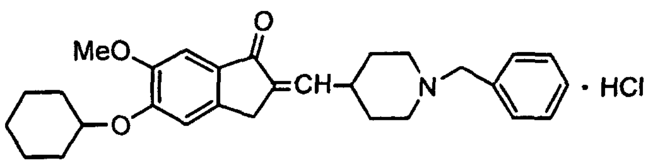

The free form of the title compound was obtained as a pale yellow oil in the same way as Example 3-3 (yield; 67%).
$^1$H-NMR (90Mz:$CDCl_3$)δ : 1.10-2.52 (19H,m), 2.72-3.02 (2H,m), 3.48 (2H,s), 3.48-3.60 (2H,m), 3.82 (3H,s), 6.52-6.78 (1H,m), 6.82 (1H,s), 7.05-7.50 (6H,m).

The product was converted into a hydrochloride in a conventional manner and recrystallized from methanol/diisopropyl ether, to give the title compound as pale yellowish white crystals.
GC-MS : m/z = 446 ($M+H^+$).

Example 20: Synthesis of 1-benzyl-4-[[5-methoxy-6-(2-propyloxy)-1-indanon]-2-ylidene]methylpiperidine hydrochloride

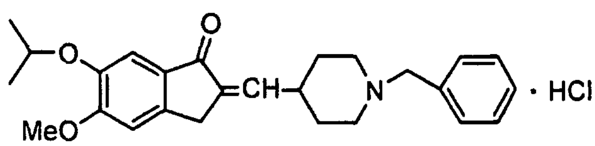

The free form of the title compound was obtained as a pale yellow oil in the same way as Example 3-3 (yield;

60%).
$^1$H-NMR (90Mz:$CDCl_3$)$\delta$ : 1.35 (3H,s), 1.41 (3H,s), 1.58-2.50 (9H,m), 2.78-3.02 (2H,m), 3.48 (2H,s), 3.48-3.60 (2H, m), 3.90 (3H,s), 4.40-4.70 (1H,m), 6.48-6.68 (1H,m), 6.81 (1H,s), 7.10-7.38 (6H,m).

The product was converted into a hydrochloride in a conventional manner and recrystallized from methanol/ diisopropyl ether, to give the title compound as pale yellowish white crystals.
EI-MS : m/z = 405 ($M^+$).

Example 21: Synthesis of 1-benzyl-4-[[5,6-(1,2-ethylenedioxy)-1-indanon]-2-yl]methylpiperidine hydrochloride

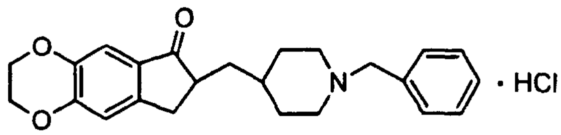

1.20 g of 1-benzyl-4-[[5,6-(1,2-ethylenedioxy)-1-indanon]-2-ylidene]methylpiperidine was dissolved in 30 ml of THF, and 0.2 g of 10% palladium/carbon was added thereto, followed by hydrogenation at room temperature at normal pressure for 2 hours. After filtering off the catalyst, the filtrate was evaporated. The resulting residue was purified by silica gel column chromatography (methylene chloride/methanol system), to give 0.59 g (87%) of the free form of the title compound as a pale yellow oil.
$^1$H-NMR (90Mz:$CDCl_3$)$\delta$ : 1.08-3.35 (14H,m), 3.48 (2H,s), 4.10-4.35 (4H,m), 6.80 (1H,s), 7.18 (1H,s), 7.18-7.32 (5H,m).

The product was converted into a hydrochloride in a conventional manner and recrystallized from ethanol/ diisopropyl ether, to give the title compound as pale yellowish white crystals.
GC-MS : m/z = 377 ($M^+$).

Example 22: Synthesis of 1-benzyl-4-[(5-cyclohexyloxy-1-indanon)-2-yl]methylpiperidine hydrochloride

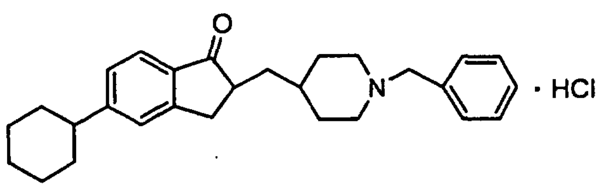

The free form of the title compound was obtained as a pale yellow oil in the same way as Example 21 (yield; 75%).
$^1$H-NMR (90Mz:$CDCl_3$)$\delta$ : 1.00-3.42 (25H,m), 3.48 (2H,s), 7.10 (1H,s), 7.10-7.68 (7H,m).

The product was converted into a hydrochloride in a conventional manner and recrystallized from ethanol/ diisopropyl ether, to give the title compound as pale yellowish white crystals.
GC-MS : m/z = 401 ($M^+$).

Example 23: Synthesis of 1-benzyl-4-[(5-cyclohexyloxy-6-methoxy-1-indanon)-2-yl]methylpiperidine hydrochloride

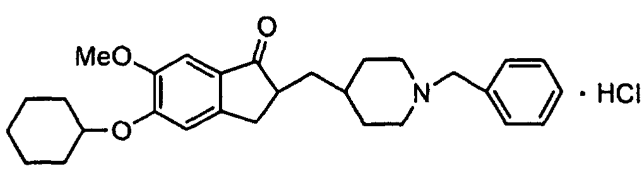

The free form of the title compound was obtained as a pale yellow oil in the same way as Example 21 (yield; 86%).
$^1$H-NMR (90Mz:$CDCl_3$)$\delta$ : 1.10-3.18 (24H,m), 3.48 (2H,s), 3.82 (3H,s), 4.10-4.45 (1H,m), 6.79 (1H,s), 7.10 (1H,s), 7.10-7.33 (5H,m).
The product was converted into a hydrochloride in a conventional manner and recrystallized from ethanol/diisopropyl ether, to give the title compound as pale yellowish white crystals.
GC-MS : m/z = 448 ($M^+$).

Example 24: Synthesis of 1-benzyl-4-[[5-methoxy-6-(2-propyloxy)-1-indanon]-2-yl]methylpiperidine hydrochloride

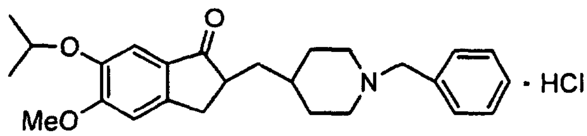

The free form of the title compound was obtained as a pale yellow oil in the same way as Example 21 (yield; 54%).
$^1$H-NMR (90Mz:$CDCl_3$)$\delta$ : 1.05-3.35 (14H,m), 1.35 (3H,s), 1.40 (3H,s), 3.48 (2H,s), 3.88 (3H,s), 4.35-4.70 (1H,m), 6.78 (1H,s), 7.10 (1H,s), 7.10-7.35 (5H,m).
The product was converted into a hydrochloride in a conventional manner and recrystallized from methanol/diisopropyl ether, to give the title compound as pale yellowish white crystals.
GC-MS : m/z = 408 ($M^+$).

Example 25: 1-Benzyl-4-[(5,6-dimethoxy-1-indanon)-2-ylidene]methylpiperidine hydrochloride

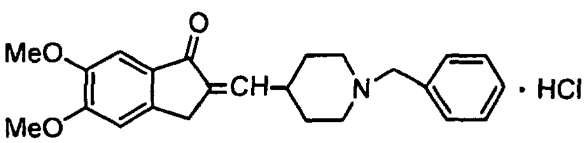

The title compound described in JP-A 2-169569 was found to have a sigma receptor binding action.

Example 26: 1-Benzyl-4-[(5,6-diethoxy-1-indanon)-2-ylidene]methylpiperidine hydrochloride

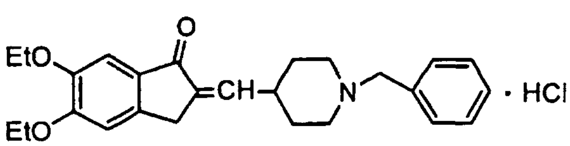

The title compound described in JP-A 2-169569 was found to have a sigma receptor binding action.

Example 27: 1-Benzyl-4-[(1-indanon)-2-yl]methylpiperidine hydrochloride

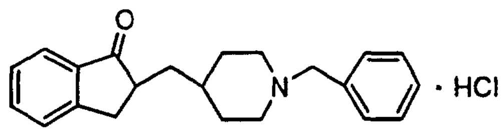

The title compound described in JP-A 2-169569 was found to have a sigma receptor binding action.

Example 28: 1-Benzyl-4-[(5-methoxy-1-indanon)-2-yl]methylpiperidine hydrochloride

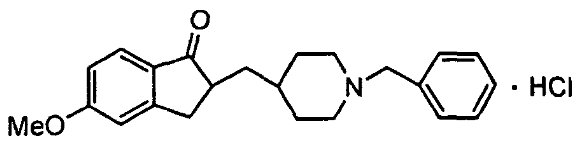

The title compound described in JP-A 2-169569 was found to have a sigma receptor binding action.

Example 29: 1-Benzyl-4-[(5-ethoxy-6-methoxy-1-indanon)-2-yl]methylpiperidine hydrochloride

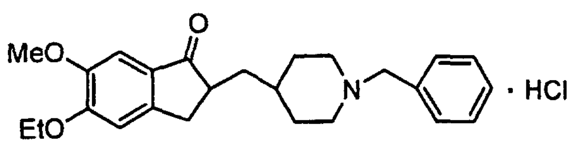

The title compound described in JP-A 2001-139547 was found to have a sigma receptor binding action.

Example 30: 1-Benzyl-4-[(5,6-diethoxy-1-indanon)-2-yl]methylpiperidine hydrochloride

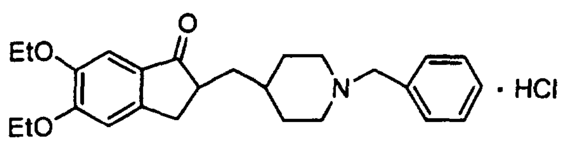

The title compound described in JP-A 2-169569 was found to have a sigma receptor binding action.

Example 31: 1-Benzyl-4-[[5,6-di(1-propyloxy)-1-indanon)-2-yl]methylpiperidine hydrochloride

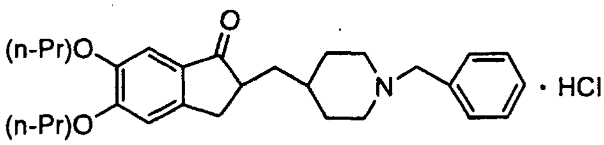

The title compound described in JP-A 2000-319258 was found to have a sigma receptor binding action.

Example 32: 1-Benzyl-4-[2-[(5,6-dimethoxy-1-indanon)-2-yl]ethyl]piperidine hydrochloride

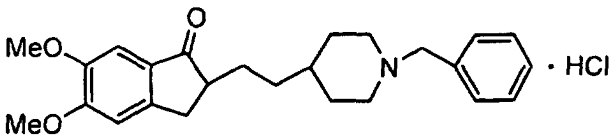

The title compound described in JP-A 2-169569 was found to have a sigma receptor binding action.

Example 33: 1-Benzyl-4-[3-[(5,6-dimethoxy-1-indanon)-2-yl]propyl]piperidine hydrochloride

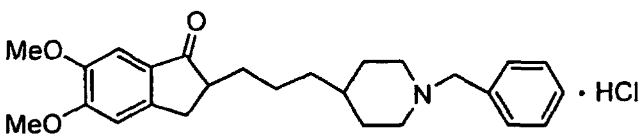

The title compound described in JP-A 2-169569 was found to have a sigma receptor binding action.

Example 34: 1-(3-Fluorobenzyl)-4-[(5,6-dimethoxy-1-indanon)-2-yl]methylpiperidine hydrochloride

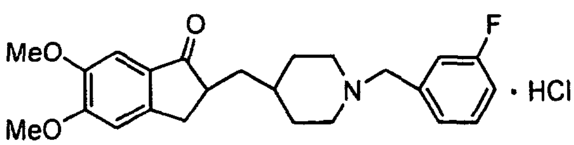

The title compound described in JP-A 2-169569 was found to have a sigma receptor binding action.

Example 35: 1-(3-Methylbenzyl)-4-[(5,6-dimethoxy-1-indanon)-2-yl]methylpiperidine hydrochloride

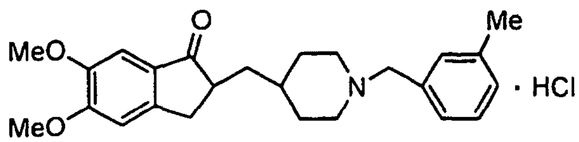

The title compound described in JP-A 2-169569 was found to have a sigma receptor binding action.

Example 36: 1-Cyclohexylmethyl-4-[(5,6-dimethoxy-1-indanon)-2-yl]methylpiperidine hydrochloride

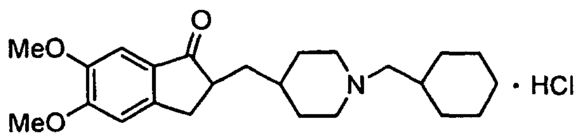

The title compound described in JP-A 2-169569 was found to have a sigma receptor binding action.

Example 37: Synthesis of 1-benzyl-4-[(6-ethoxy-2-fluoro-5-methoxy-1-indanon)-2-yl]methylpiperidine hydrochloride

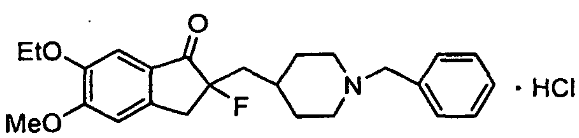

The following reactions were performed under nitrogen atmosphere.

0.14 g of 1-benzyl-4-[(6-ethoxy-5-methoxy-1-indanon)-2-yl]methylpiperidine was dissolved in 7 ml of tetrahydrofuran (THF), cooled to -78°C, and then 0.53 ml of a 1.0 M solution of lithium bis(trimethylsilyl)amide in THF was poured thereinto. After the temperature of the mixture was elevated from -78°C to -10°C over 30 minutes, it was cooled again to -78°C, and a solution of 0.17 g of N-fluorobenzenesulfonimide in 3 ml of THF was poured thereinto. The temperature of the mixture was gradually elevated from -78°C to room temperature. After stirring for 5 hours, a saturated aqueous solution of ammonium chloride was added thereto and the mixture was extracted with ethyl acetate. The organic layer was washed with brine, dried over magnesium sulfate ($MgSO_4$), and evaporated. The resulting residue was purified by silica gel column chromatography (NH-silica gel; n-hexane/ethyl acetate system), to give 70 mg (48%) of the free form of the title compound as a pale yellow oil.

$^1$H-NMR (400Mz:$CDCl_3$)δ : 1.35-1.45 (2H,m), 1.48 (3H,t,*J*=6.8Hz), 1.58-1.77 (4H,m), 1.90-2.08 (3H,m), 2.80-2.88 (2H, m), 3.26 (1H,dd,*J*=16.8Hz,*J*=24Hz), 3.30 (1H,s), 3.47 (2H,s), 3.96 (3H,s), 4.12 (2H,q,*J*=6.8Hz), 6.82 (1H,s), 7.18 (1H, s), 7.21-7.33 (5H,m).

The product was converted into a hydrochloride in a conventional manner and recrystallized from ethanol/t-butyl methyl ether, to give the title compound as pale yellowish white crystals.

ESI-MS : m/z = 412 ($M+H^+$).

Example 38: Synthesis of 1-benzyl-4-[(5-ethoxy-2-fluoro-6-methoxy-1-indanon)-2-yl]methylpiperidine hydrochloride

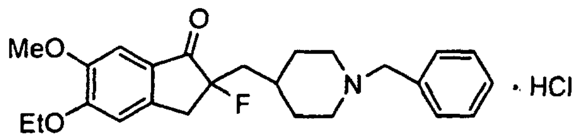

The free form of the title compound was obtained as a pale yellow oil in the same way as Example 37 (yield; 48%).
$^1$H-NMR (400Mz:$CDCl_3$)δ : 1.35-1.45 (2H,m), 1.53 (3H,t,*J*=6.8Hz), 1.58-1.77 (4H,m), 1.90-2.08 (3H,m), 2.80-2.88 (2H, m), 3.25 (1H,dd,*J*=16Hz,*J*=24Hz), 3.29 (1H,s), 3.47 (2H,s), 3.90 (3H,s), 4.19 (2H,q,*J*=6.8Hz), 6.80 (1H,s), 7.19 (1H, s), 7.22-7.33 (5H,m).
The product was converted into a hydrochloride in a conventional manner and recrystallized from ethanol/t-butyl methyl ether, to give the title compound as pale yellowish white crystals.
ESI-MS : m/z = 412 (M+H$^+$).

Example 39: Synthesis of 1-benzyl-4-[[2-fluoro-6-methoxy-5-(1-propyloxy)-1-indanon-2-yl]methylpiperidine hydrochloride

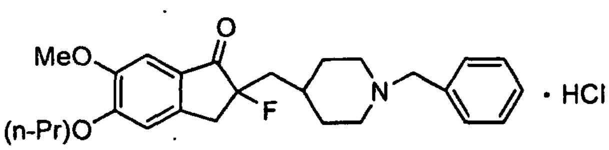

The free form of the title compound was obtained as a pale yellow oil in the same way as Example 37 (yield; 80%).
$^1$H-NMR (400Mz:$CDCl_3$)δ : 1.07 (3H,t,*J*=6.8Hz), 1.35-1.46 (2H,m), 1.59-1.77 (4H,m), 1.87-2.08 (5H,m), 2.80-2.87 (2H, m), 3.25 (1H,dd,*J*=16Hz,*J*=24Hz), 3.29 (1H,s), 3.47 (2H,s), 3.89 (3H,s), 4.06 (2H,t,*J*=6.8Hz), 6.80 (1H,s), 7.18 (1H,s), 7.22-7.32 (5H,m).
The product was converted into a hydrochloride in a conventional manner and recrystallized from ethanol/t-butyl methyl ether, to give the title compound as pale yellowish white crystals.
ESI-MS : m/z = 426 (M+H$^+$).

Example 40: Synthesis of 1-(2-fluorobenzyl)-4-[(5,6-diethoxy-2-fluoro-1-indanon)-2-yl]methylpiperidine hydrochloride

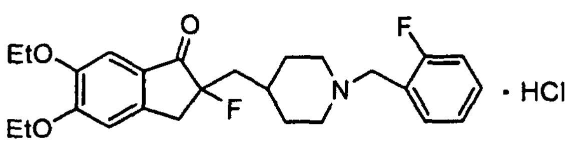

The free form of the title compound was obtained as a pale yellow oil in the same way as Example 37 (yield; 67%).
$^1$H-NMR (400Mz:$CDCl_3$)δ : 1.35-1.45 (2H,m), 1.47 (3H,t,*J*=6.8Hz), 1.52 (3H,t,*J*=6.8Hz), 1.57-1.79 (4H,m), 1.96-2.07 (3H,m), 2.82-2.90 (2H,m), 3.24 (1H,dd,*J*=16Hz,*J*=24Hz), 3.28 (1H,s), 3.55 (2H,s), 4.11 (2H,q,*J*=6.8Hz), 4.18 (2H,q,

$J$=6.8Hz), 6.79 (1H,s), 6.98-7.04 (1H,m), 7.07-7.12 (1H,m), 7.17 (1H,s), 7.19-7.26 (1H,m), 7.36 (1H,dt,$J$=1.6Hz, $J$=7.6Hz).

The product was converted into a hydrochloride in a conventional manner and recrystallized from ethanol/t-butyl methyl ether, to give the title compound as pale yellowish white crystals.
ESI-MS : m/z = 444 ($M+H^+$).

Example 41: Synthesis of 1-(3-fluorobenzyl)-4-[(5,6-diethoxy-2-fluoro-1-indanon)-2-yl]methylpiperidine hydrochloride

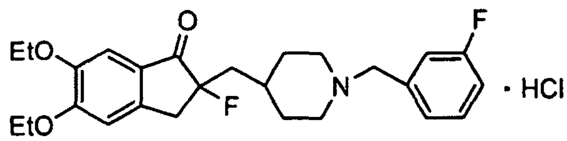

The free form of the title compound was obtained as a pale yellow oil in the same way as Example 37 (yield; 53%).
$^1$H-NMR (400Mz:$CDCl_3$)δ : 1.34-1.46 (2H,m), 1.47 (3H,t,$J$=6.8Hz), 1.52 (3H,t,$J$=6.8Hz), 1.59-1.78 (4H,m), 1.92-2.09 (3H,m), 2.79-2.88 (2H,m), 3.25 (1H,dd,$J$=16Hz,$J$=24Hz), 3.29 (1H,s), 3.46 (2H,s), 4.11 (2H,q,$J$=6.8Hz), 4.18 (2H,q, $J$=6.8Hz), 6.80 (1H,s), 6.89-6.96 (1H,m), 7.02-7.09 (2H,m), 7.18 (1H,s), 7.22-7.29 (1H,m).

The product was converted into a hydrochloride in a conventional manner and recrystallized from ethanol/t-butyl methyl ether, to give the title compound as pale yellowish white crystals.
ESI-MS : m/z = 444 ($M+H^+$).

Example 42: Synthesis of 1-(4-fluorobenzyl)-4-[(5,6-diethoxy-2-fluoro-1-indanon)-2-yl]methylpiperidine hydrochloride

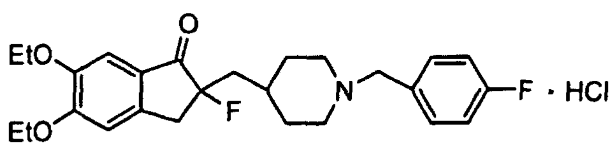

The free form of the title compound was obtained as a pale yellow oil in the same way as Example 37 (yield; 74%). $^1$H-NMR (400Mz:$CDCl_3$)δ : 1.33-1.42 (2H,m), 1.47 (3H,t,$J$=6.8Hz), 1.52 (3H,t,$J$=6.8Hz), 1.57-1.77 (4H,m), 1.88-2.08 (3H,m), 2.77-2.84 (2H,m), 3.27 (1H,dd,$J$=16Hz,$J$=24Hz), 3.29 (1H,s), 3.42 (2H,s), 4.11 (2H,q,$J$=6.8Hz), 4.18 (2H,q,$J$=6.8Hz), 6.80 (1H,s), 6.95-7.02 (2H,m), 7.18 (1H,s), 7.22-7.27 (2H,m).

The product was converted into a hydrochloride in a conventional manner and recrystallized from ethanol/t-butyl methyl ether, to give the title compound as pale yellowish white crystals.
ESI-MS : m/z = 444 ($M+H^+$).

Example 43: Synthesis of 1-(2-chlorobenzyl)-4-[(5,6-diethoxy-2-fluoro-1-indanon)-2-yl]methylpiperidine hydrochloride

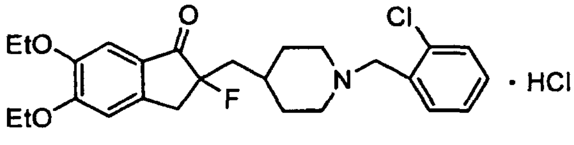

The free form of the title compound was obtained as a pale yellow oil in the same way as Example 37 (yield; 54%).

$^1$H-NMR (400Mz:$CDCl_3$) δ: 1.37-1.47 (2H,m), 1.47 (3H,t,$J$=6.8Hz), 1.52 (3H,t,$J$=6.8Hz), 1.59-1.80 (4H,m), 1.99-2.13 (3H,m), 2.84-2.92 (2H,m), 3.26 (1H,dd,$J$=16Hz,$J$=24Hz), 3.30 (1H,s), 3.58 (2H,s), 4.11 (2H,q,$J$=6.8Hz), 4.18 (2H,q, $J$=6.8Hz), 6.80 (1H,s), 7.14-7.25 (2H,m), 7.18 (1H,s), 7.33 (1H,dd, $J$=1.6Hz,$J$=7.6Hz), 7.47 (1H,dt,$J$=1.6Hz,$J$=7.6Hz).

The product was converted into a hydrochloride in a conventional manner and recrystallized from ethanol/t-butyl methyl ether, to give the title compound as pale yellowish white crystals.
ESI-MS : m/z = 460 (M+H$^+$).

Example 44: Synthesis of 1-(3-chlorobenzyl)-4-[(5,6-diethoxy-2-fluoro-1-indanon)-2-yl]methylpiperidine hydrochloride

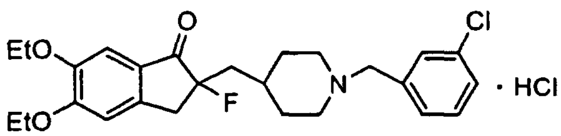

The free form of the title compound was obtained as a pale yellow oil in the same way as Example 37 (yield; 74%).
$^1$H-NMR (400Mz:$CDCl_3$)δ : 1.35-1.45 (2H,m), 1.47 (3H,t,$J$=6.8Hz), 1.52 (3H,t,$J$=6.8Hz), 1.59-1.78 (4H,m), 1.90-2.08 (3H,m), 2.77-2.86 (2H,m), 3.25 (1H,dd,$J$=16Hz,$J$=24Hz), 3.29 (1H,s), 3.43 (2H,s), 4.11 (2H,q,$J$=6.8Hz), 4.18 (2H,q, $J$=6.8Hz), 6.80 (1H,s), 7.16-7.33 (4H,m), 7.18 (1H,s).

The product was converted into a hydrochloride in a conventional manner and recrystallized from ethanol/t-butyl methyl ether, to give the title compound as pale yellowish white crystals.
ESI-MS : m/z = 460 (M+H$^+$).

Example 45: Synthesis of 1-(4-chlorobenzyl)-4-[(5,6-diethoxy-2-fluoro-1-indanon)-2-yl)methylpiperidine hydrochloride

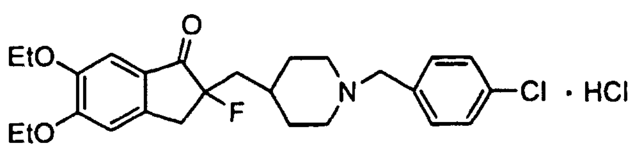

The free form of the title compound was obtained as a pale yellow oil in the same way as Example 37 (yield; 70%).
$^1$H-NMR (400Mz:$CDCl_3$)δ : 1.33-1.43 (2H,m), 1.47 (3H,t,$J$=6.8Hz), 1.52 (3H,t,$J$=6.8Hz), 1.60-1.77 (4H,m), 1.89-2.07 (3H,m), 2.76-2.84 (2H,m), 3.24 (1H,dd,$J$=16Hz,$J$=24Hz), 3.29 (1H,s), 3.42 (2H,s), 4.11 (2H,q,$J$=6.8Hz), 4.18 (2H,q, $J$=6.8Hz), 6.80 (1H,s), 7.18 (1H,s), 7.21-7.28 (4H,m).

The product was converted into a hydrochloride in a conventional manner and recrystallized from ethanol/t-butyl methyl ether, to give the title compound as pale yellowish white crystals.
ESI-MS : m/z = 460 (M+H$^+$).

Example 46: Synthesis of 1-(2-methylbenzyl)-4-[(5,6-diethoxy-2-fluoro-1-indanon)-2-yl]methylpiperidine hydrochloride

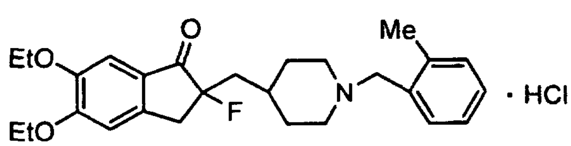

The free form of the title compound was obtained as a pale yellow oil in the same way as Example 37 (yield; 70%).
$^1$H-NMR (400Mz:$CDCl_3$)δ : 1.31-1.40 (2H,m), 1.47 (3H,t,*J*=6.8Hz), 1.52 (3H,t,*J*=6.8Hz), 1.58-1.76 (4H,m), 1.91-2.08 (3H,m), 2.34 (3H,s), 2.78-2.86 (2H,m), 3.25 (1H,dd,*J*=16Hz,*J*=24Hz), 3.29 (1H,s), 3.40 (2H,s), 4.11 (2H,q,*J*=6.8Hz), 4.18 (2H,q,*J*=6.8Hz), 6.80 (1H,s), 7.10-7.16 (3H,m), 7.18 (1H,s), 7.22-7.27 (1H,m).
The product was converted into a hydrochloride in a conventional manner and recrystallized from ethanol/t-butyl methyl ether, to give the title compound as pale yellowish white crystals.
ESI-MS : m/z = 440 (M+H$^+$).

Example 47: Synthesis of 1-(3-methylbenzyl)-4-[(5,6-diethoxy-2-fluoro-1-indanon)-2-yl]methylpiperidine hydrochloride

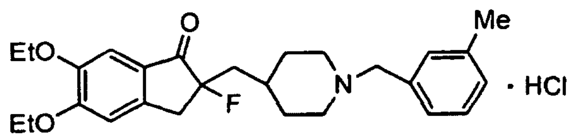

The free form of the title compound was obtained as a pale yellow oil in the same way as Example 37 (yield; 77%).
$^1$H-NMR (400Mz:$CDCl_3$)δ : 1.35-1.45 (2H,m), 1.47 (3H,t,*J*=6.8Hz), 1.52 (3H,t,*J*=6.8Hz), 1.57-1.78 (4H,m), 1.89-2.08 (3H,m), 2.34 (3H,s), 2.80-2.88 (2H,m), 3.24 (1H,dd,*J*=16Hz,*J*=24Hz), 3.29 (1H,s), 3.43 (2H,s), 4.11 (2H,q,*J*=6.8Hz), 4.18 (2H,q,*J*=6.8Hz), 6.80 (1H,s), 7.04-7.14 (3H,m), 7.18 (1H,s), 7.19 (1H,t,*J*=7.2Hz).
The product was converted into a hydrochloride in a conventional manner and recrystallized from ethanol/t-butyl methyl ether, to give the title compound as pale yellowish white crystals.
ESI-MS : m/z = 440 (M+H$^+$).

Example 48: Synthesis of 1-(4-methylbenzyl)-4-[(5,6-diethoxy-2-fluoro-1-indanon)-2-yl]methylpiperidine hydrochloride

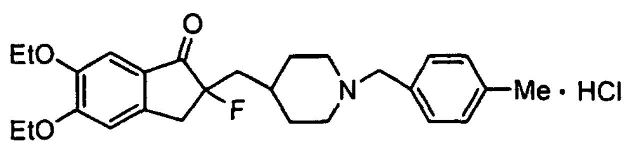

The free form of the title compound was obtained as a pale yellow oil in the same way as Example 37 (yield; 74%).
$^1$H-NMR (400Mz:$CDCl_3$)δ : 1.34-1.43 (2H,m), 1.47 (3H,t,*J*=6.8Hz), 1.52 (3H,t,*J*=6.8Hz), 1.57-1.77 (4H,m), 1.88-2.07 (3H,m), 2.33 (3H,s), 2.79-2.87 (2H,m), 3.26 (1H,dd,*J*=16Hz,*J*=24Hz), 3.28 (1H,s), 3.43 (2H,s), 4.11 (2H,q,*J*=6.8Hz), 4.18 (2H,q,*J*=6.8Hz), 6.79 (1H,s), 7.14 (4H,dd,*J*=8Hz, *J*=27.2Hz), 7.17 (1H,s).
The product was converted into a hydrochloride in a conventional manner and recrystallized from ethanol/t-butyl methyl ether, to give the title compound as pale yellowish white crystals.
ESI-MS : m/z = 440 (M+H$^+$).

Example 49: Synthesis of 1-cyclopentylmethyl-4-[(5,6-diethoxy-2-fluoro-1-indanon)-2-yl]methylpiperidine hydrochloride

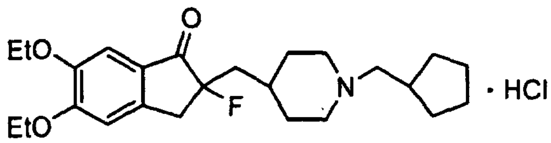

The free form of the title compound was obtained as a pale yellow oil in the same way as Example 37 (yield; 32%).
$^1$H-NMR (400Mz:$CDCl_3$)δ : 1.12-1.22 (2H,m), 1.39-1.81 (12H,m), 1.48 (3H,t,*J*=6.8Hz), 1.52 (3H,t,*J*=6.8Hz), 1.91-2.12 (4H,m), 2.29 (2H,d,*J*=7.2Hz), 2.89-2.98 (2H,m), 3.26 (1H,dd,*J*=16Hz,*J*=24Hz), 3.30 (1H,s), 4.11 (2H,q,*J*=6.8Hz), 4.19 (2H,q,*J*=6.8Hz), 6.81 (1H,s), 7.18 (1H,s).
The product was converted into a hydrochloride in a conventional manner and recrystallized from ethanol/t-butyl methyl ether, to give the title compound as pale yellowish white crystals.
ESI-MS : m/z = 418 ($M+H^+$).

Example 50: Synthesis of 1-cyclohexylmethyl-4-[(5,6-diethoxy-2-fluoro-1-indanon)-2-yl]methylpiperidine hydrochloride

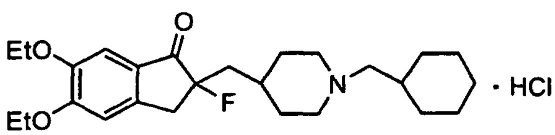

The free form of the title compound was obtained as a pale yellow oil in the same way as Example 37 (yield; 64%).
$^1$H-NMR (400Mz:$CDCl_3$)δ : 0.78-0.90 (2H,m), 1.09-1.27 (3H,m), 1.31-1.78 (12H,m), 1.47 (3H,t,*J*=6.8Hz), 1.52 (3H,t,*J*=6.8Hz), 1.82 (2H,t,*J*=11.6Hz ), 1.97-2.05 (1H,m), 2.06 (2H,d,*J*=7.2Hz), 2.76-2.84 (2H,m), 3.25 (1H,dd,*J*=17.2Hz, *J*=26Hz), 3.30 (1H,s), 4.11 (2H,q,*J*=6.8Hz), 4.18 (2H,q,*J*=6.8Hz), 6.80 (1H,s), 7.18 (1H,s).
The product was converted into a hydrochloride in a conventional manner and recrystallized from ethanol/t-butyl methyl ether, to give the title compound as pale yellowish white crystals.
ESI-MS : m/z = 432 ($M+H^+$).

Example 51: Synthesis of 1-cycloheptylmethyl-4-[(5,6-diethoxy-2-fluoro-1-indanon)-2-yl]methylpiperidine hydrochloride

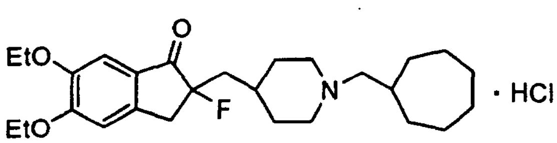

The free form of the title compound was obtained as a pale yellow oil in the same way as Example 37 (yield; 33%).
$^1$H-NMR (400Mz:$CDCl_3$)δ : 1.05-1.16 (2H,m), 1.34-1.78 (17H,m), 1.47 (3H,t,*J*=6.8Hz), 1.52 (3H,t,*J*=6.8Hz), 1.89 (2H, t,*J*=11.2Hz ), 1.96-2.08 (1H,m), 2.09 (2H,d,*J*=7.2Hz), 2.80-2.90 (2H,m), 3.26 (1H,dd,*J*=16Hz,*J*=24Hz), 3.30 (1H,s), 4.11 (2H,q,*J*=6.8Hz), 4.19 (2H,q,J=6.8Hz), 6.81 (1H,s), 7.18 (1H,s).

The product was converted into a hydrochloride in a conventional manner and recrystallized from ethanol/t-butyl methyl ether, to give the title compound as pale yellowish white crystals.
ESI-MS : m/z = 446 ($M+H^+$).

Example 52: Synthesis of 1-cyclooctylmethyl-4-[(5,6-diethoxy-2-fluoro-1-indanon)-2-yl]methylpiperidine hydrochloride

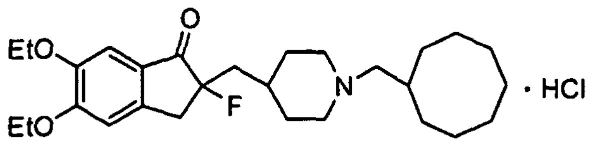

The free form of the title compound was obtained as a pale yellow oil in the same way as Example 37 (yield; 69%).
$^1$H-NMR (400Mz:$CDCl_3$)δ : 1.13-1.22 (2H,m), 1.30-1.76 (20H,m), 1.47 (3H,t,*J*=6.8Hz), 1.52 (3H,t,*J*=6.8Hz), 1.79-1.87 (2H,m), 2.01 (2H,d,*J*=7.2Hz), 2.76-2.83 (2H,m), 3.26 (1H,dd,*J*=16Hz,*J*=28Hz), 3.30 (1H,d,*J*=2.8Hz), 4.11 (2H,q,*J*=6.8Hz), 4.19 (2H,q,*J*=6.8Hz), 6.80 (1H,s), 7.18 (1H,s).
The product was converted into a hydrochloride in a conventional manner and recrystallized from ethanol/t-butyl methyl ether, to give the title compound as pale yellowish white crystals.
ESI-MS : m/z = 460 ($M+H^+$).

Example 53: Synthesis of 1-benzyl-4-[(5-cyanomethoxy-2-fluoro-6-methoxy-1-indanon)-2-yl]methylpiperidine hydrochloride

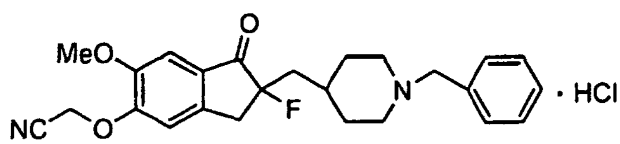

53-1) 1-Benzyl-4-[[5-(t-butyl)dimethylsilyloxy-6-methoxy-1-indanon]-2-yl]methylpiperidine

0.50 g of 1-benzyl-[(5-hydroxy-6-methoxy-1-indanon)-2-yl]methylpiperidine described in JP-A 2-169569 was dissolved in 10 ml of dimethylformamide (DMF), followed by addition of 0.23 g of imidazole and 0.41 g of (t-butyl) dimethylchlorosilane. After stirring at room temperature overnight, water was added thereto and the mixture was extracted with ethyl acetate. The organic layer was washed with brine, dried over magnesium sulfate ($MgSO_4$), and evaporated. The resulting residue was purified by silica gel column chromatography (methylene chloride/methanol system), to give 0.51 g (78%) of the title compound.
$^1$H-NMR (400Mz:$CDCl_3$)δ : 0.89 (6H,s), 0.91 (9Hs), 1.15-1.45 (3H,m), 1.53-1.74 (3H,m), 1.77-1.92 (3H,m), 2.52-2.62 (2H,m), 2.76-2.84 (2H,m), 3.09 (1H,dd,*J*=7.2Hz,*J*=16.8Hz), 3.40 (2H,s), 3.73 (3H,s), 6.75 (1H,s), 7.06 (1H,s), 7.13-7.25 (5H,m).

53-2) 1-Benzyl-4-[(2-fluoro-5-hydroxy-6-methoxy-1-indanon)-2-yl]methylpiperidine

0.50 g of the above obtained 1-benzyl-4-[[5-(t-butyl)dimethylsilyloxy-6-methoxy-1-indanon]-2-yl]methylpiperidine was treated in the same way as Example 1, to give an unpurified fluorine derivative.
The product was dissolved in 12 ml of THF, followed by addition of 1.15 ml of a 1.0 M solution of tetrabutylammonium fluoride in THF. After stirring at room temperature for 30 minutes, the mixture was evaporated. The resulting residue was purified by silica gel column chromatography and further by preparative thin layer silica gel column chromatography (methylene chloride/methanol system), to give 0.39 g (total yield in the two steps; 97%) of the title compound.
$^1$H-NMR (400Mz:$CDCl_3$)δ : 1.40-1.54 (2H,m), 1.58-1.80 (4H,m), 1.97-2.11 (3H,m), 2.90-2.98 (2H,m), 3.22 (1H,dd,

$J$=16Hz,$J$=20Hz), 3.26 (2H,s), 3.91 (3H,s), 6.86 (1H,s), 7.20 (1H,s), 7.22-7.36 (5H,m).
ESI-MS : m/z = 384 (M+H$^+$).

53-3) 1-Benzyl-4-[(5-cyanomethoxy-2-fluoro-6-methoxy-1-indanon)-2-yl]methylpiperidine

0.15 g of 1-benzyl-4-[(2-fluoro-5-hydroxy-6-methoxy-1-indanon)-2-yl]methylpiperidine was dissolved in 4 ml of DMF, followed by addition of 39 mg of a 60% sodium hydride under ice-cooling. After stirring for 30 minutes, 0.068 ml of acetonitrile bromide was added thereto and the mixture was stirred at room temperature for 5 hours. A saturated aqueous solution of ammonium chloride was added thereto, and the mixture was extracted with ethyl acetate. The extract was washed with brine, dried over magnesium sulfate ($MgSO_4$), and evaporated. The resulting residue was purified by preparative thin layer silica gel column chromatography (methylene chloride/methanol system), to give 21 mg (13%) of the title compound.
$^1$H-NMR (400Mz:$CDCl_3$)$\delta$ : 1.37-1.48 (2H,m), 1.60-1.79 (4H,m), 1.94-2.05 (3H,m), 2.83-2.91 (2H,m), 3.30 (1H,dd, $J$=16Hz,$J$=28Hz), 3.34 (1H,s), 3.50 (2H,s), 3.92 (3H,s), 4.93 (2H,dd,$J$=16Hz,$J$=20Hz), 7.00 (1H,s), 7.22-7.33 (5H,m), 7.27 (1H,s).

The product was converted into a hydrochloride in a conventional manner and then freeze-dried, to give the title compound as an amorphous.
ESI-MS : m/z = 423 (M+H$^+$).

Example 54: 1-Benzyl-4-[(2-fluoro-1-indanon)-2-yl]methylpiperidine hydrochloride

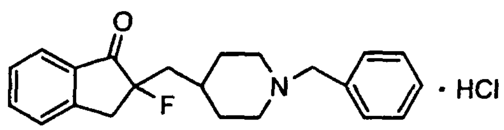

The title compound described in JP-A 2000-319258 was found to have a sigma receptor binding action.

Example 55: 1-Benzyl-4-[(5-methoxy-2-fluoro-1-indanon)-2-yl]methylpiperidine hydrochloride

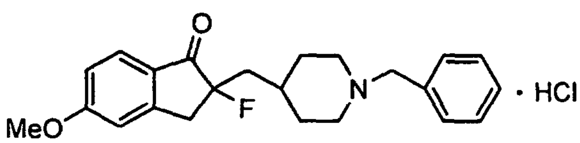

The title compound described in JP-A 2000-319258 was found to have a sigma receptor binding action.

Example 56: 1-Benzyl-4-[(5,6-dimethoxy-2-fluoro-1-indanon)-2-yl]methylpiperidine hydrochloride

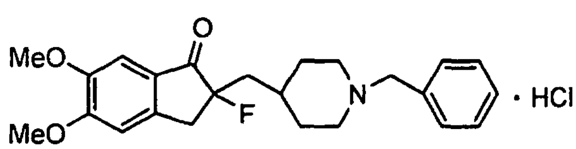

The title compound described in JP-A 2000-319257 was found to have a sigma receptor binding action.

Example 57: 1-Benzyl-4-[(5,6-diethoxy-2-fluoro-1-indanon)-2-yl]methylpiperidine hydrochloride

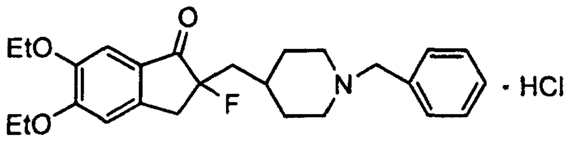

The title compound described in JP-A 2000-319258 was found to have a sigma receptor binding action.

Example 58: 1-Benzyl-4-[(5,6-di(1-propyloxy)-2-fluoro-1-indanon]-2-yl]methylpiperidine hydrochloride

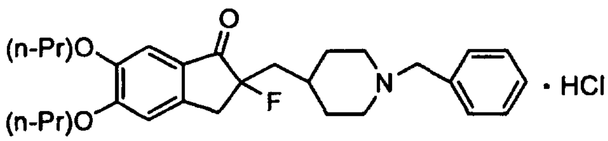

The title compound described in JP-A 2000-319258 was found to have a sigma receptor binding action.

Example 59: 1-Benzyl-4-[(5,6-dimethoxy-2-fluoro-1-indanon)-2-yl]piperidine

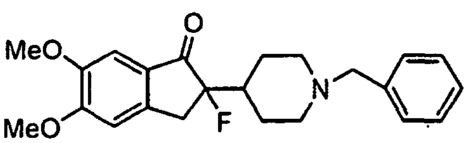

The title compound described in JP-A 2000-319258 was found to have a sigma receptor binding action.

Example 60: 1-Benzyl-4-[2-[(5,6-dimethoxy-2-fluoro-1-indanon)-2-yl]ethyl]piperidine

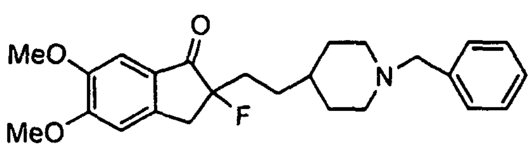

The title compound described in JP-A 2000-319258 was found to have a sigma receptor binding action.

Example 61: 1-Benzyl-4-[3-[(5,6-dimethoxy-2-fluoro-1-indanon)-2-yl]propyl]piperidinepiperidine hydrochloride

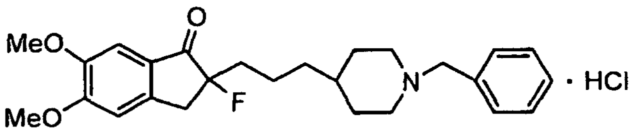

The title compound described in JP-A 2000-319258 was found to have a sigma receptor binding action.

Example 62: 1-(2-Fluorobenzyl)-4-[(5,6-dimethoxy-2-fluoro-1-indanon)-2-yl]methylpiperidine hydrochloride

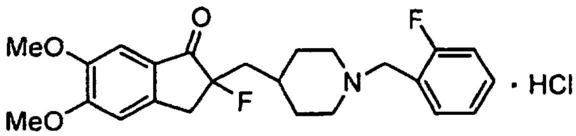

The title compound described in JP-A 2000-319258 was found to have a sigma receptor binding action.

Example 63: 1-(3-Fluorobenzyl)-4-[(5,6-dimethoxy-2-fluoro-1-indanon)-2-yl]methylpiperidine hydrochloride

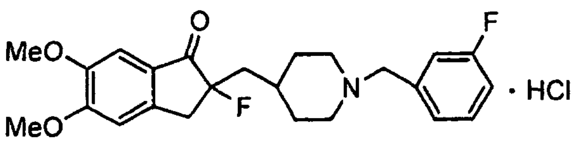

The title compound described in JP-A 2000-319258 was found to have a sigma receptor binding action.

Example 64: 1-(4-Fluorobenzyl)-4-[(5,6-dimethoxy-2-fluoro-1-indanon)-2-yl]methylpiperidine hydrochloride

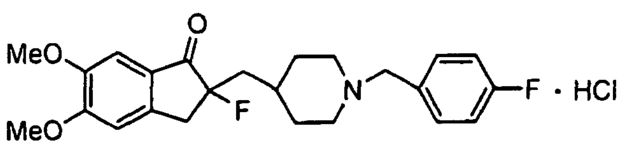

The title compound described in JP-A 2000-319258 was found to have a sigma receptor binding action.

Example 65: 1-(3-Methylbenzyl)-4-[(5,6-dimethoxy-2-fluoro-1-indanon)-2-yl]methylpiperidine hydrochloride

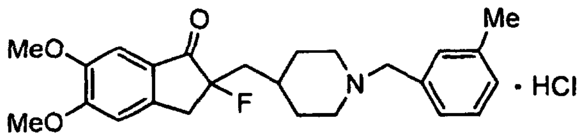

The title compound described in JP-A 2000-319258 was found to have a sigma receptor binding action.

Example 66: 1-Cyclohexylmethyl-4-[(5,6-dimethoxy-2-fluoro-1-indanon)-2-yl]methylpiperidine hydrochloride

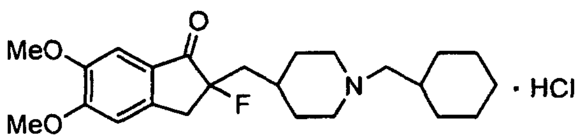

The title compound described in JP-A 2000-319258 was found to have a sigma receptor binding action.

Example 67: 1-Benzyl-4-[(5,6-dimethoxy-2-chloro-1-indanon)-2-yl]methylpiperidine hydrochloride

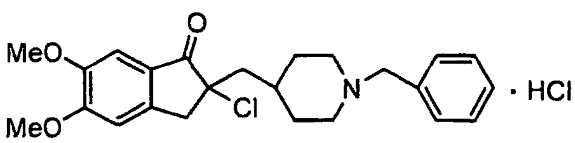

The title compound described in JP-A 2001-139547 was found to have a sigma receptor binding action.

Example 68: 1-Benzyl-4-[(5,6-diethoxy-2-chloro-1-indanon)-2-yl]methylpiperidine hydrochloride

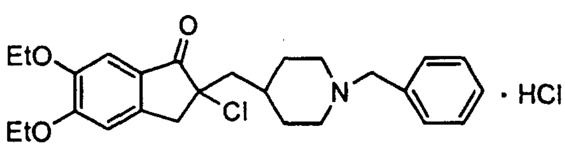

The title compound described in JP-A 2001-139547 was found to have a sigma receptor binding action.

Example 69: 1-Benzyl-4-[(5-ethoxy-6-methoxy-2-chloro-1-indanon)-2-yl]methylpiperidine hydrochloride

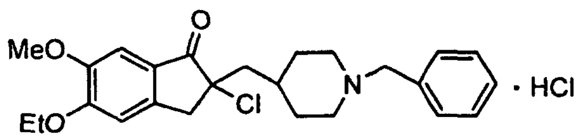

The title compound described in JP-A 2001-139547 was found to have a sigma receptor binding action.

Example 70: 1-Benzyl-4-[(5,6-dimethoxy-2-bromo-1-indanon)-2-yl]methylpiperidine hydrochloride

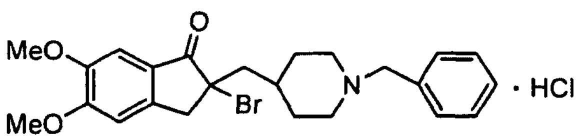

The title compound described in JP-A 2001-139547 was found to have a sigma receptor binding action.

Example 71: 1-Benzyl-4-[(5,6-dimethoxy-2-methyl-1-indanon)-2-yl]methylpiperidine hydrochloride

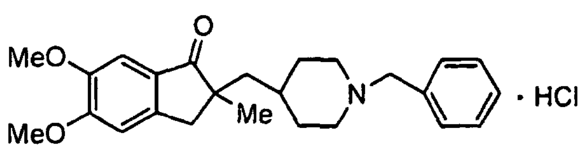

The title compound described in JP-A 2001-139547 was found to have a sigma receptor binding action.

Example 72: Synthesis of 1-(3,4-difluorobenzyl)-4-[(5,6-diethoxy-1-indanon)-2-yl]methylpiperidine hydrochloride

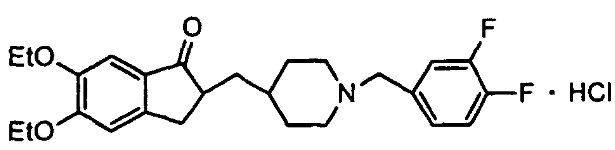

The free form of the title compound was obtained as a pale yellow oil in the same way as Example 4 (yield; 69%).

$^1$H-NMR (400Mz:$CDCl_3$)δ : 1.23-1.54 (4H,m), 1.46 (3H,t,*J*=7Hz), 1.51 (3H,t,*J*=7Hz), 1.63-1.77 (2H,m), 1.87-2.00 (3H, m), 2.64-2.72 (2H,m), 2.80-2.89 (2H,m), 3.21 (1H,dd,*J*=8Hz,*J*=18Hz), 3.43 (2H,s), 4.11 (2H,q,*J*=7Hz), 4.17 (2H,q, *J*=7Hz), 6.83 (1H,s), 6.98-7.20 (3H,m),, 7.16 (1H,s).

The product was converted into a hydrochloride in a conventional manner and recrystallized from ethanol, to

give the title compound as pale yellowish white crystals.
EI-MS : m/z = 444 (M+H$^+$).

Example 73: Synthesis of 1-(3,5-difluorobenzyl)-4-[(5,6-diethoxy-1-indanon)-2-yl]methylpiperidine hydrochloride

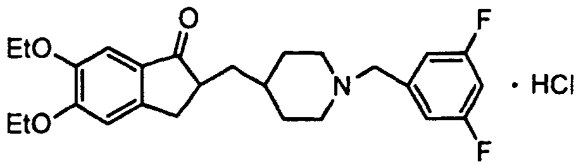

The free form of the title compound was obtained as a pale yellow oil in the same way as Example 4 (yield; 83%).
$^1$H-NMR (400Mz:$CDCl_3$)δ : 1.27-1.54 (4H,m), 1.47 (3H,t,*J*=7Hz), 1.51 (3H,t,J=7Hz), 1.64-1.78 (2H,m), 1.88-2.04 (3H, m), 2.64-2.73 (2H,m), 2.81-2.90 (2H,m), 3.22 (1H,dd,*J*=8Hz,*J*=18Hz), 3.46 (2H,s), 4.11 (2H,q,*J*=7Hz), 4.17 (2H,q, *J*=7Hz), 6.63-6.72 (1H,m), 6.83 (1H,s), 6.85-6.92 (2H,m), 7.16 (1H,s).
The product was converted into a hydrochloride in a conventional manner and recrystallized from ethanol, to give the title compound as pale yellowish white crystals.
EI-MS : m/z = 444 (M+H$^+$).

Example 74: Synthesis of 1-(3,4-difluorobenzyl)-4-[(5,6-diethoxy-2-fluoro-1-indanon)-2-yl]methylpiperidine hydrochloride

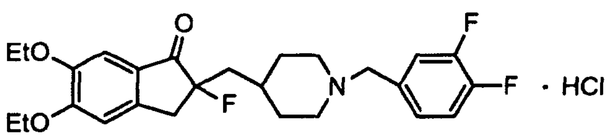

The free form of the title compound was obtained as a pale yellow oil in the same way as Example 37 (yield; 42%).
$^1$H-NMR (400Mz:$CDCl_3$)δ : 1.33-1.44 (2H,m), 1.47 (3H,t,*J*=7Hz), 1.52 (3H,t,*J*=7Hz), 1.59-1.77 (4H,m), 1.90-2.07 (3H, m), 2.75-2.83 (2H,m), 3.25 (1H,dd,*J*=18Hz,*J*=23Hz), 3.29 (1H,s), 3.40 (2H,s), 4.11 (2H,q,*J*=7Hz), 4.18 (2H,q,*J*=7Hz), 6.60 (1H,s), 6.97-7.19 (3H,m), 7.18 (1H,s).
The product was converted into a hydrochloride in a conventional manner and recrystallized from ethanol/t-butyl methyl ether, to give the title compound as pale yellowish white crystals.
EI-MS : m/z = 462 (M+H$^+$).

Example 75: Synthesis of 1-(3,5-difluorobenzyl)-4-[(5,6-diethoxy-2-fluoro-1-indanon)-2-yl]methylpiperidine hydrochloride

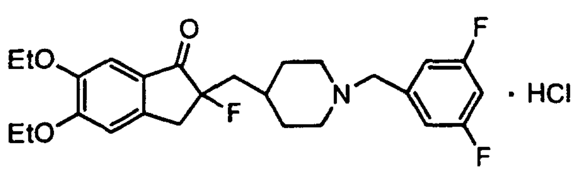

The free form of the title compound was obtained as a pale yellow oil in the same way as Example 37 (yield;

54%).
$^1$H-NMR (400Mz:$CDCl_3$)δ : 1.35-1.44 (2H,m), 1.47 (3H,t,*J*=7Hz), 1.52 (3H,t,*J*=7Hz), 1.60-1.77 (4H,m), 1.92-2.08 (3H, m), 2.76-2.84 (2H,m), 3.25 (1H,dd,*J*=18Hz, *J*=23Hz), 3.29 (1H,s), 3.42 (2H,s), 4.11 (2H,q,*J*=7Hz), 4.18 (2H,q,*J*=7Hz), 6.66 (1H,tt,*J*=2Hz,*J*=9Hz), 6.80 (1H,s), 6.82-6.88 (2H,m), 7.18 (1H,s).

The product was converted into a hydrochloride in a conventional manner and recrystallized from ethanol/t-butyl methyl ether, to give the title compound as pale yellowish white crystals.
EI-MS : m/z = 462 ($M+H^+$).

## Claims

**1.** A sigma receptor binding agent comprising an indanone compound represented by the following formula (I), a pharmacologically acceptable salt thereof or a hydrate of them.

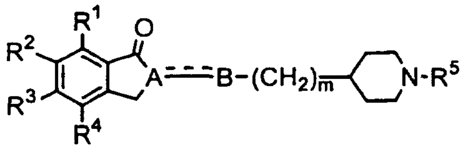

(I)

In the formula (I), $R^1$, $R^2$, $R^3$ and $R^4$ are the same as or different from each other and each represents hydrogen atom, a halogen atom, hydroxyl group, nitrile group, a $C_{1-6}$ alkyl group which may be substituted, a cycloalkyl group having three to eight carbon atoms which may be substituted, a $C_{1-6}$ alkoxy group which may be substituted, a cycloalkoxy group having three to eight carbon atoms which may be substituted, an acyl group having one to six carbon atoms which may be substituted, a $C_{1-6}$ alkoxycarbonyl group which may be substituted, a $C_{1-6}$ alkylaminocarbonyloxy group which may be substituted, a di($C_{1-6}$ alkyl)aminocarbonyloxy group which may be substituted, nitro group, an amino group which may be substituted, an amide group which may be substituted, mercapto group or a thio-$C_{1-6}$ alkoxy group which may be substituted, and further $R^1$ with $R^2$, $R^2$ with $R^3$, or $R^3$ with $R^4$ may together form an aliphatic ring, an aromatic ring, a heterocyclic ring or an alkylenedioxy ring; the partial structure:

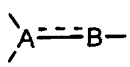

represents a group represented by $>CH-CH_2-$, $>C=CH-$ or $>C(-R^7)-CH_2-$; m represents an integer of 0 or 1 to 5; and $R^5$ represents hydrogen atom, a $C_{1-6}$ alkyl group which may be substituted, a $C_{2-6}$ alkenyl group which may be substituted, a $C_{2-6}$ alkynyl group which may be substituted, a cycloalkyl group having three to eight carbon atoms which may be substituted, a 2,2-(alkylenedioxy)ethyl group or a group represented by the formula:

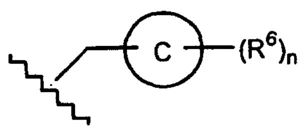

(wherein the ring C represents benzene ring, an aliphatic ring or a heterocyclic ring; $R^6$s are the same as or different from each other and each represents hydrogen atom, a halogen atom, hydroxyl group, nitrile group, a $C_{1-6}$ alkyl group which may be substituted, a $C_{2-6}$ alkenyl group which may be substituted, a $C_{2-6}$ alkynyl group which may be substituted, a cycloalkyl group having three to eight carbon atoms which may be substituted, a $C_{1-6}$ alkoxy group which may be substituted, a $C_{1-6}$ alkoxyalkoxy group which may be substituted, an aryloxy group which may be substituted or an aralkyloxy group which may be substituted, and further two of $R^6$s may together form an aliphatic ring, an aromatic ring, a heterocyclic ring or an alkylenedioxy ring; $R^7$ represents a halogen atom, hydroxyl group, a $C_{1-6}$ alkyl group, a $C_{1-6}$ alkoxy group, nitrile group, a halogeno-$C_{1-6}$ alkyl group, a hydroxyl-$C_{1-6}$ alkyl group, a cyano-$C_{1-6}$ alkyl group, an amino-$C_{1-6}$ alkyl group, nitro group, azide group, an amino group which may be substituted, carbamoyl group which may be substituted, carboxyl group which may be substituted, mercapto

group or a thio-$C_{1-6}$ alkoxy group; and n represents an integer of 1 to 5), provided that 1-benzyl-4-[(5,6-dimethoxy-1-indanon)-2-yl]methylpiperidine, a pharmacologically acceptable salt thereof or a hydrate of them are excluded.

**2.** The sigma receptor binding agent comprising an indanone compound, a pharmacologically acceptable salt thereof or a hydrate of them according to claim 1, wherein the indanone compound represented by the formula (I) is one selected from:

(1) 1-benzyl-4-[(5,6-dimethoxy-1-indanon)-2-ylidene]methylpiperidine,
(2) 1-benzyl-4-[(5,6-diethoxy-1-indanon)-2-ylidene]methylpiperidine,
(3) 1-benzyl-4-[(1-indanon)-2-yl]methylpiperidine,
(4) 1-benzyl-4-[(5-methoxy-1-indanon)-2-yl]methylpiperidine,
(5) 1-benzyl-4-[(5-ethoxy-6-methoxy-1-indanon)-2-yl]methylpiperidine,
(6) 1-benzyl-4-[(5,6-diethoxy-1-indanon)-2-yl]methylpiperidine,
(7) 1-benzyl-4-[[5,6-di(1-propyloxy)-1-indanon)-2-yl]methylpiperidine,
(8) 1-benzyl-4-[2-[(5,6-dimethoxy-1-indanon)-2-yl]ethyl]piperidine,
(9) 1-benzyl-4-[3-[(5,6-dimethoxy-1-indanon)-2-yl]propyl]piperidine,
(10) 1-(3-fluorobenzyl)-4-[(5,6-dimethoxy-1-indanon)-2-yl]methylpiperidine,
(11) 1-(3-methylbenzyl)-4-[(5,6-dimethoxy-1-indanon)-2-yl]methylpiperidine,
(12) 1-cyclohexylmethyl-4-[(5,6-dimethoxy-1-indanon)-2-yl]methylpiperidine,
(13) 1-benzyl-4-[(2-fluoro-1-indanon)-2-yl]methylpiperidine,
(14) 1-benzyl-4-[(5-methoxy-2-fluoro-1-indanon)-2-yl]methylpiperidine,
(15) 1-benzyl-4-[(5,6-dimethoxy-2-fluoro-1-indanon)-2-yl]methylpiperidine,
(16) 1-benzyl-4-[(5,6-diethoxy-2-fluoro-1-indanon)-2-yl]methylpiperidine,
(17) 1-benzyl-4-[[5,6-di(1-propyloxy)-2-fluoro-1-indanon]-2-yl]methylpiperidine,
(18) 1-benzyl-4-[(5,6-dimethoxy-2-fluoro-1-indanon)-2-yl]piperidine,
(19) 1-benzyl-4-[2-[(5,6-dimethoxy-2-fluoro-1-indanon)-2-yl]ethyl]piperidine,
(20) 1-benzyl-4-[3-[(5,6-dimethoxy-2-fluoro-1-indanon)-2-yl]propyl]piperidine,
(21) 1-(2-fluorobenzyl)-4-[(5,6-dimethoxy-2-fluoro-1-indanon)-2-yl]methylpiperidine,
(22) 1-(3-fluorobenzyl)-4-[(5,6-dimethoxy-2-fluoro-1-indanon)-2-yl]methylpiperidine,
(23) 1-(4-fluorobenzyl)-4-[(5,6-dimethoxy-2-fluoro-1-indanon)-2-yl]methylpiperidine,
(24) 1-(3-methylbenzyl)-4-[(5,6-dimethoxy-2-fluoro-1-indanon)-2-yl]methylpiperidine,
(25) 1-cyclohexylmethyl-4-[(5,6-dimethoxy-2-fluoro-1-indanon)-2-yl]methylpiperidine,
(26) 1-benzyl-4-[(5,6-dimethoxy-2-chloro-1-indanon)-2-yl]methylpiperidine,
(27) 1-benzyl-4-[(5,6-diethoxy-2-chloro-1-indanon)-2-yl]methylpiperidine,
(28) 1-benzyl-4-[(5-ethoxy-6-methoxy-2-chloro-1-indanon)-2-yl]methylpiperidine,
(29) 1-benzyl-4-[(5,6-dimethoxy-2-bromo-1-indanon)-2-yl]methylpiperidine, and
(30) 1-benzyl-4-[(5,6-dimethoxy-2-methyl-1-indanon)-2-yl]methylpiperidine.

**3.** The sigma receptor binding agent according to claim 1 or 2, which is a sigma receptor antagonist or a sigma receptor agonist.

**4.** The sigma receptor binding agent according to claim 1 or 2, which is an agent for preventing, treating or improving a disease against which a sigma receptor agonistic drug is efficacious.

**5.** The sigma receptor binding agent according to claim 1 or 2, which is an agent for preventing, treating or improving a disease against which a sigma receptor antagonistic action is efficacious.

**6.** The sigma receptor binding agent according to claim 1 or 2, which is an agent for preventing, treating or improving a disease against which a sigma receptor agonistic action is efficacious.

**7.** The sigma receptor binding agent according to claim 1 or 2, which is an agent for preventing, treating or improving a mental disorder.

**8.** The sigma receptor binding agent according to claim 7, wherein the mental disorder is at least one selected from a disorder accompanied with cerebrovascular dementia and/or senile dementia, schizophrenia, emotional disorder, depression, neurosis, psychophysiologic disorder and anxiety.

**9.** The sigma receptor binding agent according to claim 8, wherein the disorder accompanied with cerebrovascular

dementia and/or senile dementia is at least one selected from aggressive behavior, mental excitement, wandering, delirium, hallucination and hyperkinesis.

**10.** The sigma receptor binding agent according to claim 1 or 2, which is an agent for improving intellectual function.

**11.** The indanone compound represented by the formula (I) according to claim 1, a pharmacologically acceptable salt thereof or a hydrate of them, wherein the indanone compound is one selected from:

(1) 1-benzyl-4-[[5,6-(1,2-ethylenedioxy)-1-indanon]-2-ylidene]methylpiperidine,
(2) 1-benzyl-4-[(5-cyclohexyl-1-indanon)-2-ylidene]methylpiperidine,
(3) 1-benzyl-4-[(5-cyclohexyloxy-6-methoxy-1-indanon)-2-ylidene]methylpiperidine,
(4) 1-benzyl-4-[[5-methoxy-6-(2-propyloxy)-1-indanon]-2-ylidene]methylpiperidine,
(5) 1-benzyl-4-[[5,6-(1,2-ethylenedioxy)-1-indanon]-2-yl]methylpiperidine,
(6) 1-benzyl-4-[[5,6-cyclohexyl-1-indanon]-2-yl]methylpiperidine,
(7) 1-benzyl-4-[(5-cyclohexyloxy-6-methoxy-1-indanon)-2-yl]methylpiperidine,
(8) 1-benzyl-4-[[5-methoxy-6-(2-propyloxy)-1-indanon]-2-yl]methylpiperidine,
(9) 1-benzyl-4-[(6-ethoxy-5-methoxy-1-indanon)-2-yl]methylpiperidine,
(10) 1-benzyl-4-[[6-methoxy-5-(1-propyloxy)-1-indanon]-2-yl]methylpiperidine,
(11) 1-benzyl-4-[(5-cyanomethoxy-6-methoxy-1-indanon)-2-yl]methylpiperidine,
(12) 1-cyclopentylmethyl-4-[(5,6-diethoxy-1-indanon)-2-yl]methylpiperidine,
(13) 1-cyclohexylmethyl-4-[(5,6-diethoxy-1-indanon)-2-yl]methylpiperidine,
(14) 1-cycloheptylmethyl-4-[(5,6-diethoxy-1-indanon)-2-yl]methylpiperidine,
(15) 1-cyclooctylmethyl-4-[(5,6-diethoxy-1-indanon)-2-yl]methylpiperidine,
(16) 1-(2-fluorobenzyl)-4-[(5,6-diethoxy-1-indanon)-2-yl]methylpiperidine,
(17) 1-(3-fluorobenzyl)-4-[(5,6-diethoxy-1-indanon)-2-yl]methylpiperidine,
(18) 1-(4-fluorobenzyl)-4-[(5,6-diethoxy-1-indanon)-2-yl]methylpiperidine,
(19) 1-(2-chlorobenzyl)-4-[(5,6-diethoxy-1-indanon)-2-yl]methylpiperidine,
(20) 1-(3-chlorobenzyl)-4-[(5,6-diethoxy-1-indanon)-2-yl]methylpiperidine,
(21) 1-(4-chlorobenzyl)-4-[(5,6-diethoxy-1-indanon)-2-yl]methylpiperidine,
(22) 1-(2-methylbenzyl)-4-[(5,6-diethoxy-1-indanon)-2-yl]methylpiperidine,
(23) 1-(3-methylbenzyl)-4-[(5,6-diethoxy-1-indanon)-2-yl]methylpiperidine,
(24) 1-(4-methylbenzyl)-4-[(5,6-diethoxy-1-indanon)-2-yl]methylpiperidine,
(25) 1-benzyl-4-[(6-ethoxy-5-methoxy-2-fluoro-1-indanon)-2-yl]methylpiperidine,
(26) 1-benzyl-4-[(5-ethoxy-6-methoxy-2-fluoro-1-indanon)-2-yl]methylpiperidine,
(27) 1-benzyl-4-[[6-methoxy-5-(1-propyloxy)-2-fluoro-1-indanon]-2-yl]methylpiperidine,
(28) 1-(2-fluorobenzyl)-4-[(5,6-diethoxy-2-fluoro-1-indanon)-2-yl]methylpiperidine,
(29) 1-(3-fluorobenzyl)-4-[(5,6-diethoxy-2-fluoro-1-indanon)-2-yl]methylpiperidine,
(30) 1-(4-fluorobenzyl)-4-[(5,6-diethoxy-2-fluoro-1-indanon)-2-yl]methylpiperidine,
(31) 1-(2-chlorobenzyl)-4-[(5,6-diethoxy-2-fluoro-1-indanon)-2-yl]methylpiperidine,
(32) 1-(3-chlorobenzyl)-4-[(5,6-diethoxy-2-fluoro-1-indanon)-2-yl]methylpiperidine,
(33) 1-(4-chlorobenzyl)-4-[(5,6-diethoxy-2-fluoro-1-indanon)-2-yl]methylpiperidine,
(34) 1-(2-methylbenzyl)-4-[(5,6-diethoxy-2-fluoro-1-indanon)-2-yl]methylpiperidine,
(35) 1-(3-methylbenzyl)-4-[(5,6-diethoxy-2-fluoro-1-indanon)-2-yl]methylpiperidine,
(36) 1-(4-methylbenzyl)-4-[(5,6-diethoxy-2-fluoro-1-indanon)-2-yl]methylpiperidine,
(37) 1-cyclopentylmethyl-4-[(5,6-diethoxy-2-fluoro-1-indanon)-2-yl]methylpiperidine,
(38) 1-cyclohexylmethyl-4-[(5,6-diethoxy-2-fluoro-1-indanon)-2-yl]methylpiperidine,
(39) 1-cycloheptylmethyl-4-[(5,6-diethoxy-2-fluoro-1-indanon)-2-yl]methylpiperidine,
(40) 1-cyclooctylmethyl-4-[(5,6-diethoxy-2-fluoro-1-indanon)-2-yl]methylpiperidine,
(41) 1-benzyl-4-[(5-cyanomethoxy-6-methoxy-2-fluoro-1-indanon)-2-yl]methylpiperidine,
(42) 1-(3,4-difluorobenzyl)-4-[(5,6-diethoxy-1-indanon)-2-yl]methylpiperidine,
(43) 1-(3,5-difluorobenzyl)-4-[(5,6-diethoxy-1-indanon)-2-yl]methylpiperidine,
(44) 1-(3,4-difluorobenzyl)-4-[(5,6-diethoxy-2-fluoro-1-indanon)-2-yl]methylpiperidine, and
(45) 1-(3,5-difluorobenzyl)-4-[(5,6-diethoxy-2-fluoro-1-indanon)-2-yl]methylpiperidine.

**12.** A pharmaceutical composition comprising the indanone compound according to claim 11, a pharmacologically acceptable salt thereof or a hydrate of them.

**13.** The pharmaceutical composition according to claim 12, which is a sigma receptor binding agent.

**14.** The pharmaceutical composition according to claim 12, which is a sigma receptor antagonist or a sigma receptor agonist.

**15.** The pharmaceutical composition according to claim 12, which is an agent for preventing, treating or improving a disease against which a sigma receptor-active drug is efficacious.

**16.** The pharmaceutical composition according to claim 12, which is an agent for preventing, treating or improving a disease against which a sigma receptor antagonistic action is efficacious.

**17.** The pharmaceutical composition according to claim 12, which is an agent for preventing, treating or improving a disease against which a sigma receptor agonistic action is efficacious.

**18.** The pharmaceutical composition according to claim 12, which is an agent for preventing, treating or improving a mental disorder.

**19.** The pharmaceutical composition according to claim 12, wherein the mental disorder is at least one selected from a disorder accompanied with cerebrovascular dementia and/or senile dementia, schizophrenia, emotional disorder, depression, neurosis, psychosomatic disorder and anxiety.

**20.** The pharmaceutical composition according to claim 19, wherein the disorder accompanied with cerebrovascular dementia and/or senile dementia is at least one selected from aggressive behavior, mental excitement, wandering, delirium, hallucination and hyperkinesis.

**21.** The pharmaceutical composition according to claim 12, which is an agent for improving intellectual function.

**22.** The pharmaceutical composition according to claim 12, which is an acetylcholinesterase inhibitor.

**23.** The pharmaceutical composition according to claim 12, which is an agent for preventing, treating or improving senile dementia, cerebrovascular dementia, attention-deficit hyperactivity disorder, glaucoma, myasthenia gravis or migraine.

**24.** The pharmaceutical composition according to claim 23, wherein the senile dementia is Alzheimer-type dementia.

**25.** A method for preventing, treating or improving a disease against which a sigma receptor binding action is efficacious, which comprises administering a pharmacologically effective amount of the indanone compound represented by the formula (I) according to claim 1, a pharmacologically acceptable salt thereof or a hydrate of them to a patient.

**26.** Use of the indanone compound represented by the formula (I) according to claim 1, a pharmacologically acceptable salt thereof or a hydrate of them, for producing an agent for preventing, treating or improving a disease against which a sigma receptor antagonistic action is efficacious.

**27.** A method for preventing, treating or improving a disease against which a sigma receptor binding action is efficacious, which comprises administering a pharmacologically effective amount of the indanone compound according to claim 11, a pharmacologically acceptable salt thereof or a hydrate of them to a patient.

**28.** Use of the indanone compound according to claim 11, a pharmacologically acceptable salt thereof or a hydrate of them, for producing an agent for preventing, treating or improving a disease against which a sigma receptor antagonistic action is efficacious.

## INTERNATIONAL SEARCH REPORT

International application No.
PCT/JP03/00553

A. CLASSIFICATION OF SUBJECT MATTER
Int.Cl[7] A61K31/445, A61P25/00, 25/18, 25/22, 25/24, 25/28, 25/06, 27/06, 43/00, 21/04, C07D211/32

According to International Patent Classification (IPC) or to both national classification and IPC

B. FIELDS SEARCHED

Minimum documentation searched (classification system followed by classification symbols)
Int.Cl[7] A61K31/445, A61P25/00, 25/18, 25/22, 25/24, 25/28, 25/06, 27/06, 43/00, 21/04, C07D211/32

Documentation searched other than minimum documentation to the extent that such documents are included in the fields searched

Electronic data base consulted during the international search (name of data base and, where practicable, search terms used)
REGISTRY(STN), CAPLUS(STN), CAOLD(STN), MEDLINE(STN), BIOSIS(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | JP 2-169569 A (Eisai Co., Ltd.),<br>29 June, 1990 (29.06.90),<br>Full text<br>(Family: none) | 1-3,7-10,11,<br>12,18-24<br>4-6,13-17,<br>26,28 |
| X<br><br>Y | EP 296560 A2 (Eisai Co., Ltd.),<br>28 December, 1988 (28.12.88),<br>Full text<br>& JP 2733203 B2 & AU 8818216 A<br>& NO 8802696 A & DK 8803379 A<br>& FI 8802716 A & ZA 8804338 A<br>& PT 87783 A & CN 1030752 A<br>& US 4895841 A | 1-3,7-10,11,<br>12,18-24<br>4-6,13-17,<br>26,28 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 April, 2003 (03.04.03) | 22 April, 2003 (22.04.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/00553

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | US 6277866 B1 (Eisai Co., Ltd.),<br>21 August, 2001 (21.08.01),<br>Full text<br>& JP 2000-319257 A & WO 02/20482 A1 | 1-3,7-10,11,<br>12,18-24<br>4-6,13-17,<br>26,28 |
| X<br><br>Y | EP 1157989 A1 (Eisai Co., Ltd.),<br>28 November, 2001 (28.11.01),<br>Full text<br>& JP 2000-319258 A & WO 00/51985 A1<br>& AU 200028256 A & KR 2001102440 A | 1-3,7-10,11,<br>12,18-24<br>4-6,13-17,<br>26,28 |
| Y | KATO, Koki et al., TAK-147, an acetylcholinesterase inhibitor, increases choline acetyltransferase activity in cultured rat septal cholinergic neurons, Neuroscience Letters, 1999, Vol.260, pages 5 to 8, full text | 1-10,13-17,<br>26,28 |

Form PCT/ISA/210 (continuation of second sheet) (July 1998)

**INTERNATIONAL SEARCH REPORT**

International application No.
PCT/JP03/00553

**Box I Observations where certain claims were found unsearchable (Continuation of item 2 of first sheet)**

This international search report has not been established in respect of certain claims under Article 17(2)(a) for the following reasons:

1. [X] Claims Nos.: 25 and 27
because they relate to subject matter not required to be searched by this Authority, namely:
Claims 25 and 27 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
The special technical feature of claims 1-10 and 26 resides in that any of indanone derivatives represented by the formula (I) is used as a sigma receptor binder. The indanone derivatives include known substances. On the other hand, the special technical feature of claims 11-24 and 28 is considered to reside in a novel indanone derivative. There is hence no technical relationship, between the subject matter of claims 1-10 and 26 and the subject matter of claims 11-24 and 28, which involves one or more identical or corresponding special technical features.

1. [ ] As all required additional search fees were timely paid by the applicant, this international search report covers all searchable claims.

2. [X] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)